(19) Europäisches Patentamt
European Patent Office
Office européen des brevets

(11) **EP 3 360 480 A1**

(12) **EUROPEAN PATENT APPLICATION**
published in accordance with Art. 153(4) EPC

(43) Date of publication:
**15.08.2018 Bulletin 2018/33**

(21) Application number: **16855084.6**

(22) Date of filing: **10.08.2016**

(51) Int Cl.:
*A61B 5/16* (2006.01)      *A61B 5/0484* (2006.01)
*A61B 5/055* (2006.01)      *A61B 5/048* (2006.01)

(86) International application number:
**PCT/JP2016/003712**

(87) International publication number:
**WO 2017/064826 (20.04.2017 Gazette 2017/16)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **16.10.2015   JP 2015204963
16.10.2015   JP 2015204969
10.06.2016   JP 2016116449**

(71) Applicant: **Hiroshima University
Higashi-Hiroshima-shi
Hiroshima 739-8511 (JP)**

(72) Inventors:
• **KANAYAMA, Noriaki
Hiroshima-shi
Hiroshima 734-8553 (JP)**

• **MAKITA, Kai
Hiroshima-shi
Hiroshima 734-8553 (JP)**
• **SASAOKA, Takafumi
Hiroshima-shi
Hiroshima 734-8553 (JP)**
• **MACHIZAWA, Masahiro
Hiroshima-shi
Hiroshima 734-8553 (JP)**
• **MATSUMOTO, Tomoya
Hiroshima-shi
Hiroshima 734-8553 (JP)**
• **YAMAWAKI, Shigeto
Hiroshima-shi
Hiroshima 734-8553 (JP)**

(74) Representative: **BRP Renaud & Partner mbB
Rechtsanwälte Patentanwälte
Steuerberater
Königstraße 28
70173 Stuttgart (DE)**

(54) **SENSITIVITY EVALUATION METHOD**

(57)      Disclosed herein is quantitative evaluation of sensitivity including: extracting cerebral physiological information items respectively related to axes of a multi-axis sensitivity model from regions of interest relevant to pleasure/displeasure, activation/deactivation, and a sense of expectation, the axes including a pleasure/displeasure axis, an activation/deactivation axis, and a sense of expectation axis; and evaluating the sensitivity using the cerebral physiological information items of the axes of the multi-axis sensitivity model.

FIG. 2

MULTI-AXIS SENSITIVITY MODEL

EP 3 360 480 A1

**Description**

TECHNICAL FIELD

The present invention relates to a method for quantitatively evaluating sensitivity.

BACKGROUND ART

When a human operates an object such as a machine or a computer, he or she generally operates an auxiliary device, e.g., a handle, a lever, a button, a keyboard, or a mouse, with part of his or her body such as hands and feet, or communicates with the object through speech or gesture. Research and development has been made on a technology, which is called "Brain Machine Interface" (BMI) or "Brain Computer Interface" (BCI), of directly connecting a human's brain to a machine so that the human can operate the machine as intended. BMI or BCI is expected to improve usability of an object through direct communication between the human and the object. Also in the fields of medical care and welfare, it is expected that BMI or BCI allows people, who lost their motor function or sensory function due to an accident or disease, to operate the object at their own will so that they can communicate with other people.
In addition to the operation of the object through the human's will or active consciousness, studies has also been made to read human's unconsciousness or subconsciousness and use it for the operation of the object. For example, there has been a technology of automatically classifying target information items based on a subject's electroencephalogram data which is related to his or her subjective knowledge or sensitivity, and is generated when the subject visually recognizes the target information items (see, e.g., Patent Document 1). According to another known technology (see, e.g., Patent Document 2), internal information of an operator, such as attention or memory, is estimated or predicted based on internal information of the operator estimated from his or her vital function measurements and brain function measurements, thereby detecting or alerting that human errors are likely to occur. It has also been known that an object from which a human feels a sense of similarity is detected using information about higher cerebral activities indicating subconsciousness (see, e.g., Patent Document 3).

CITATION LIST

PATENT DOCUMENTS

[Patent Document 1] Japanese Unexamined Patent Publication No. 2003-58298
[Patent Document 2] Japanese Unexamined Patent Publication No. 2011-150408
[Patent Document 3] Japanese Unexamined Patent Publication No. 2014-115913
[Patent Document 4] Japanese Unexamined Patent Publication No. 2006-95266
[Patent Document 5] Japanese Unexamined Patent Publication No. 2011-120824
[Patent Document 6] Japanese Unexamined Patent Publication No. 2005-58449

SUMMARY OF THE INVENTION

TECHNICAL PROBLEM

If human's mental activity or information of his or her mind, such as unconsciousness or subconsciousness, in particular sensitivity, could be read, human-and mind-friendly objects and services would be provided. For example, if human's sensitivity about an object could be objectively detected or predicted, an object that would evoke such sensitivity from the human would be designed in advance. Further, the information about the sensitivity thus read can improve mental care and human-to-human communication. The present inventors aim to develop a Brain Emotion Interface (BEI) which reads the human's sensitivity, and achieves connection or communication between humans, or between humans and objects, using the read sensitivity information.
Cerebral physiological information, such as an electroencephalogram (EEG), can be effectively used for implementing the BEI. It is therefore an object of the present disclosure to quantitatively evaluate the sensitivity using the cerebral physiological information.

SOLUTION TO THE PROBLEM

A sensitivity evaluation method according to an aspect of the present disclosure includes: extracting cerebral physiological information items related to axes of a multi-axis sensitivity model from regions of interest respectively relevant to pleasure/displeasure, activation/deactivation, and a sense of expectation, the axes including a pleasure/displeasure axis, an activation/deactivation axis, and a sense-of-expectation axis; and evaluating the sensitivity using the cerebral physiological information items of the axes of the multi-axis sensitivity model.

A sensitivity evaluation method according to another aspect of the present disclosure includes: extracting cerebral physiological information items related to axes of a multi-axis sensitivity model from regions of interest respectively relevant to pleasure/displeasure, activation/deactivation, and a sense of expectation, the axes including a pleasure/displeasure axis, an activation/deactivation axis, and a sense-of-expectation axis; obtaining cerebral physiological index values ($EEG_{pleasure}$, $EEG_{activation}$, and $EEG_{sense\ of\ expectation}$) of the axes from the cerebral physiological information items of the axes of the multi-axis sensitivity model; and evaluating the sensitivity by the following formula using a subjective psychological axis which is obtained from subjective statistical data of a subject and represents weighting coefficients (a, b, c) of the axes of the multi-axis sensitivity model:

$$\text{Sensitivity} = [\text{Subjective Psychological Axis}] \times [\text{Cerebral Physiological Index}]$$

$$= a \times EEG_{pleasure} + b \times EEG_{activation} + c \times EEG_{sense\ of\ expectation}$$

A sensitivity evaluation method according to still another aspect of the present disclosure includes: obtaining electroencephalogram signals of a subject; performing an independent component analysis on the obtained electroencephalogram signals to estimate the position of a dipole for each of the independent components; performing a principal component analysis on the independent components obtained through the independent component analysis to dimensionally reduce cerebral activity data of the independent components; forming clusters of the cerebral activity data of the dimensionally reduced independent components; selecting, from the obtained clusters, a cluster representing cerebral activities respectively reflecting various feelings or emotions; calculating evaluation values of the feelings or emotions from components included in the selected cluster; and calculating an evaluation value of the sensitivity by synthesizing the calculated evaluation values of the feelings or emotions.

A sensitivity evaluation method according to yet another aspect of the present disclosure includes: obtaining BOLD signals across a whole brain of a subject by fMRI; selecting, from the obtained BOLD signals, BOLD signals in a voxel representing cerebral activities respectively reflecting various feelings or emotions; calculating evaluation values of the feelings or emotions from the selected BOLD signals in the voxel; and calculating an evaluation value of the sensitivity by synthesizing the calculated evaluation values of the feelings or emotions.

ADVANTAGES OF THE INVENTION

According to the present disclosure, sensitivity can be evaluated quantitatively using cerebral physiological information. Thus, use of the sensitivity information makes it possible to design a product which is more appealing to the sensitivity of a human and makes the human more attached to the product as he or she uses it more frequently. Alternatively, smooth human-to-human communication can be achieved via the sensitivity information.

BRIEF DESCRIPTION OF THE DRAWINGS

[FIG. 1] FIG. 1 schematically shows relationship among emotions, feelings, and sensitivity.
[FIG. 2] FIG. 2 schematically shows a multi-axis sensitivity model adopted in the present disclosure.
[FIG. 3] FIG. 3 shows regions of interest relevant to axes of the multi-axis sensitivity model.
[FIG. 4] FIG. 4 shows various fMRI images obtained when a participant is in a pleasant state.
[FIG. 5] FIG. 5 shows an fMRI image, and a sagittal section of the brain on which EEG signal sources are plotted, both of which are obtained in the pleasant state.
[FIG. 6] FIG. 6 shows a result of a time-frequency analysis that was carried out on signals of the EEG signal sources in the region of interest (posterior cingulate gyrus in the pleasant state).
[FIG. 7] FIG. 7 shows an fMRI image, and a sagittal section of the brain on which EEG signal sources are plotted, both of which were obtained when the participant is in an active state.
[FIG. 8] FIG. 8 shows a result of a time-frequency analysis that was carried out on signals of the EEG signal sources in the region of interest (posterior cingulate gyrus in the active state).
[FIG. 9] FIG. 9 generally shows how to carry out an experiment of presenting participants with pleasant/unpleasant stimulus images.

[FIG. 10] FIG. 10 shows fMRI images of a subject's brain in a pleasant image expectation state and in an unpleasant image expectation state.

[FIG. 11] FIG. 11 shows a sagittal section of the brain (a region of parietal lobe) on which plotted are signal sources corresponding to a difference between EEG signals measured in a pleasant image expectation state and in an unpleasant image expectation state, and time-frequency distributions of the EEG signals of the region.

[FIG. 12] FIG. 12 shows a sagittal section of the brain (visual cortex) on which plotted are signal sources corresponding to a difference between EEG signals measured in a pleasant image expectation state and in an unpleasant image expectation state, and time-frequency distributions of the EEG signals of the region.

[FIG. 13] FIG. 13 shows an example of self-evaluation for determining a subjective physiological axis.

[FIG. 14] FIG. 14 shows a flow chart for identification of independent components of an electroencephalogram in the region of interest and their frequency bands.

[FIG. 15] FIG. 15 shows components (electroencephalogram topographic images) representing signal intensity distributions of independent components extracted through an independent component analysis carried out on an electroencephalogram signal.

[FIG. 16] FIG. 16 shows a sagittal section of the brain on which estimated positions of the signal sources of the independent signal components are plotted.

[FIG. 17] FIG. 17 shows an fMRI image obtained in the pleasant/unpleasant state.

[FIG. 18] FIG. 18 shows a result of a time-frequency analysis carried out on signals of the EEG signal sources.

[FIG. 19] FIG. 19 shows a flow chart of real-time sensitivity evaluation using the electroencephalogram.

[FIG. 20] FIG. 20 shows components (electroencephalogram topographic images) representing signal intensity distributions of independent components extracted through an independent component analysis carried out on an electroencephalogram signal.

[FIG. 21] FIG. 21 shows the component identified as the independent component relevant to the region of interest.

[FIG. 22] FIG. 22 shows a result of a time-frequency analysis carried out on the identified independent component.

[FIG. 23] FIG. 23 schematically shows an estimated value of a pleasure/displeasure axis.

[FIG. 24] FIG. 24 shows estimated values of an activation/deactivation axis and a sense of expectation axis.

[FIG. 25] FIG. 25 shows electroencephalogram topographic images of 16 clusters.

[FIG. 26] FIG. 26 shows brain images of 16 clusters on each of which the estimated positions of dipoles are plotted.

[FIG. 27] FIG. 27 shows an electroencephalogram topographic image of a cluster which had a significant difference between pleasant stimulus presentation and unpleasant stimulus presentation, and brain images on each of which the estimated positions of dipoles are plotted.

[FIG. 28] FIG. 28 shows correlation between subjective evaluation and cerebral activities when a subject watches (a) a pleasant image and (b) an unpleasant image.

[FIG. 29] FIG. 29 shows a flow chart of a sensitivity evaluation method using an electroencephalogram according to an embodiment of the present disclosure.

[FIG. 30] FIG. 30 shows brain images in a pleasant image expectation state.

[FIG. 31] FIG. 31 shows brain images in an unpleasant image expectation state.

[FIG. 32] FIG. 32 shows a flow chart of a sensitivity evaluation method using fMRI according to an embodiment of the present disclosure.

DESCRIPTION OF EMBODIMENTS

Embodiments will be described in detail with reference to the drawings as needed. Note that excessively detailed description will sometimes be omitted herein to avoid complexity. For example, detailed description of a matter already well known in the art and redundant description of substantially the same configuration will sometimes be omitted herein. This will be done to avoid redundancies in the following description and facilitate the understanding of those skilled in the art.

Note that the present inventors provide the following detailed description and the accompanying drawings only to help those skilled in the art fully appreciate the present invention and do not intend to limit the scope of the subject matter of the appended claims by that description or those drawings.

It will be described below in this order: 1. Background to the Invention; 2. Identification of Region of Interest; 3. Visualization of Sensitivity; 4. Sensitivity Evaluation Method using Electroencephalogram; and 5. Sensitivity Evaluation Method using fMRI. The method of item 4 has been described in Japanese Patent Application No. 2015-204963 to which the present application claims priority, and the method of item 5 in Japanese Patent Application No. 2015-204969 to which the present application claims priority.

1. Background to the Invention

A human being feels a sense of excitement, exhilaration, or suspense, or feels a flutter, on seeing or hearing something or on touching something or being touched by something. It has been considered that these senses are not mere emotions or feelings but brought about by complex, higher cerebral activities in which exteroception entering the brain through a somatic nervous system including motor nerves and sensory nerves, interoception based on an autonomic nervous system including sympathetic nerves and parasympathetic nerves, memories, experiences, and other factors are deeply intertwined with each other.

The present inventors grasp these complex, higher cerebral functions such as the senses of exhilaration, suspense, and flutter, which are distinctly different from mere emotions or feeling, as "sensitivities" comprehensively. The present inventors also defines the sensitivities as a higher cerebral function of synthesizing together the exteroceptive information (somatic nervous system) and the interoceptive information (autonomic nervous system) and looking down upon an emotional reaction produced by reference to past experiences and memories from an even higher level. In other words, the "sensitivity" can be said to be a higher cerebral function allowing a person to intuitively sense the gap between his or her prediction (image) and the result (sense information) by comparing it to his or her past experiences and knowledge.

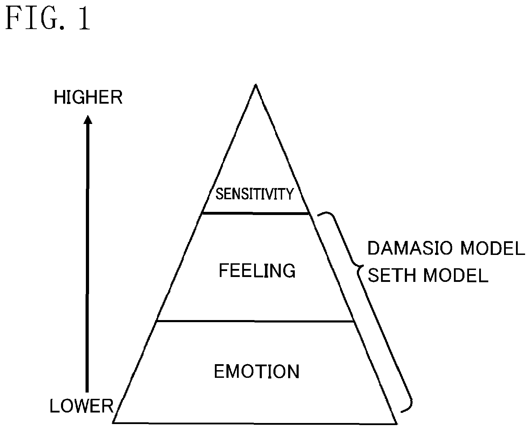

The three concepts of emotions, feelings, and sensitivity, will be described below. FIG. 1 schematically illustrates relationship among emotions, feelings, and sensitivity. The emotions are an unconscious and instinctive cerebral function caused by external stimulation, and is the lowest cerebral function among the three. The feelings are conscientized emotions, and are a higher cerebral function than the emotions. The sensitivity is a cerebral function, unique to human beings, reflective of their experiences and knowledge, and is the highest cerebral function among the three.

Viewing the sensitivity that is such a higher cerebral function in perspective requires grasping the sensitivity comprehensively from various points of view or aspects.

For example, the sensitivity may be grasped from a "pleasant/unpleasant" point of view or aspect by determining whether the person is feeling fine, pleased, or comfortable, or otherwise, feeling sick, displeased, or uncomfortable.

Alternatively, the sensitivity may also be grasped from an "active/inactive" point of view or aspect by determining whether the person is awaken, heated, or active, or otherwise, absent-minded, calm, or inactive.

Still alternatively, the sensitivity may also be grasped from a "sense of expectation" point of view or aspect by determining whether the person is excited with the expectation or anticipation of something, or otherwise, bitterly disappointed and discouraged.

A Russell's circular ring model, plotting the "pleasant/unpleasant" and "active/inactive" parameters on dual axes, is known. The feelings can be represented by this circular ring model. However, the present inventors believe that the sensitivity is a higher cerebral function of comparing the gap between the prediction (image) and the result (sense information) to experiences and knowledge, and therefore, cannot be sufficiently expressed by the traditional circular ring model comprised of the two axes indicating pleasure/displeasure and activation/deactivation. Thus, the present inventors advocate a multi-axis sensitivity model in which the time axis (indicating a sense of expectation, for example) is added as a third axis to the Russell's circular ring model.

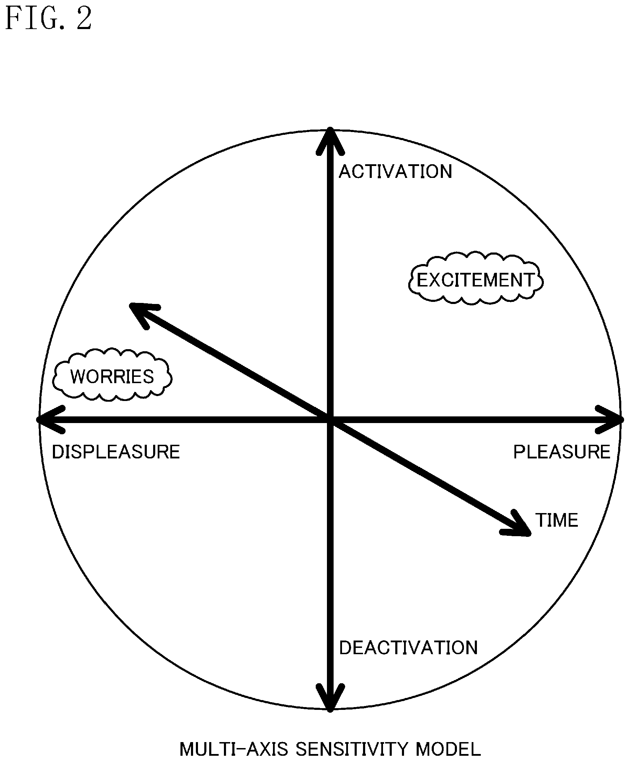

FIG. 2 schematically shows the multi-axis sensitivity model adopted in the present disclosure. The multi-axis sensitivity model can plot, for example, a "pleasant/unpleasant" parameter on a first axis, an "active/inactive" parameter on a second axis, and a "time" (sense of expectation) parameter on a third axis. Representing the sensitivity in the form of a multi-axis model is advantageous because evaluation values on these axes are calculated and synthesized so that the sensitivity, which is a vague and broad concept, can be quantitatively evaluated, or visualized.

Correct evaluation of the sensitivity, which is the higher cerebral function, would lead to establishment of the BEI technology that connects humans and objects together. If the sensitivity information is used in various fields, new values can be created, and new merits can be provided. For example, it can be considered that social implementation of the BEI technology is achieved through creation of products and systems that response more appropriately to human mind as they are used more frequently, and grow emotional values, such as pleasure, willingness, and affection.

There have been a large number of prior documents mentioning the sensitivity, all of which consider sensitivities synonymous with feelings without sharply distinguishing the former from the latter. For example, Patent Document 4 regards sensitivities as including feelings and intensions and discloses a method for quantitatively measuring the sensitivity state of a person who is happy, sad, angry, or glad, for example. However, Patent Document 4 does not distinguish sensitivities from feelings, and fails to teach evaluating the sensitivity from the time-axis (sense of expectation) point of view.

Patent Document 5 (see, in particular, claim 5) discloses a method for quantitatively evaluating a plurality of sensitivities to be pleasure, displeasure, and so forth, by making a principle component analysis on biometric information about a subject given some stimulus corresponding to pleasure, displeasure, or any other sensitivity for learning purposes. Patent Document 6, which has been cited as prior art in Patent Document 5, discloses an apparatus for visualizing feeling data as feeling parameter values using a feeling model such as a dual-axis model or a triple-axis model. However, as is clear from its description stating that sensitivity cannot be evaluated quantitatively, Patent Document 6 mixes up feelings and sensitivities, and neither teaches nor suggests the time axis (sense of expectation).

Patent Document 6 forms feeling models using a first axis indicating the degree of closeness of a person's feeling to either pleasure or displeasure, a second axis indicating the degree of closeness of his or her feeling to either an excited or tense state or a relaxed state, and a third axis indicating the degree of closeness of his or her feeling to either a tight-rope state or a slackened state, and discloses a method for expressing his or her feeling status using feeling parameters indicated as coordinate values in the three-axis space. However, these are nothing but models for expressing feelings and just a complicated version of the Russell's circular ring model.

As can be seen from the foregoing description, the techniques disclosed in these prior documents all stay within the limits of feeling analysis, and could not be used to evaluate the sensitivities properly.

2. Identification of Region of Interest

It will be described below the results of fMRI and EEG measurements performed to identify which part of the brain is active in the cerebral responses of "pleasure/displeasure," "activation/deactivation," and "sense of expectation." The measurement results are fundamental data for visualizing and quantifying the sensitivity, and thus, are of significant importance.

fMRI is one of brain function imaging methods in which a certain mental process is noninvasively associated with a specific brain structure. fMRI measures a signal intensity depending on the level of oxygen in a regional cerebral blood flow involved in neural activities. For this reason, fMRI is sometimes called a "Blood Oxygen Level Dependent" (BOLD) method.

Activities of nerve cells in the brain require a lot of oxygen. Thus, oxyhemoglobin, which is hemoglobin bonded with oxygen, flows toward the nerve cells through the cerebral blood flow. At that time, oxygen supplied exceeds the oxygen intake of the nerve cells, and as a result, reduced hemoglobin (deoxyhemoglobin) that has transported oxygen relatively decreases locally. The reduced hemoglobin has magnetic properties, and locally produces nonuniformity in the magnetic field around the blood vessel. Using hemoglobin that varies the magnetic properties depending on the bonding with oxygen, fMRI catches signal enhancement that occurs secondarily due to local change in oxygenation balance of the cerebral blood flow accompanying the activities of the nerve cells. At present, it is possible to measure in seconds the local change in the cerebral blood flow in the whole brain at a spatial resolution of about several millimeters.

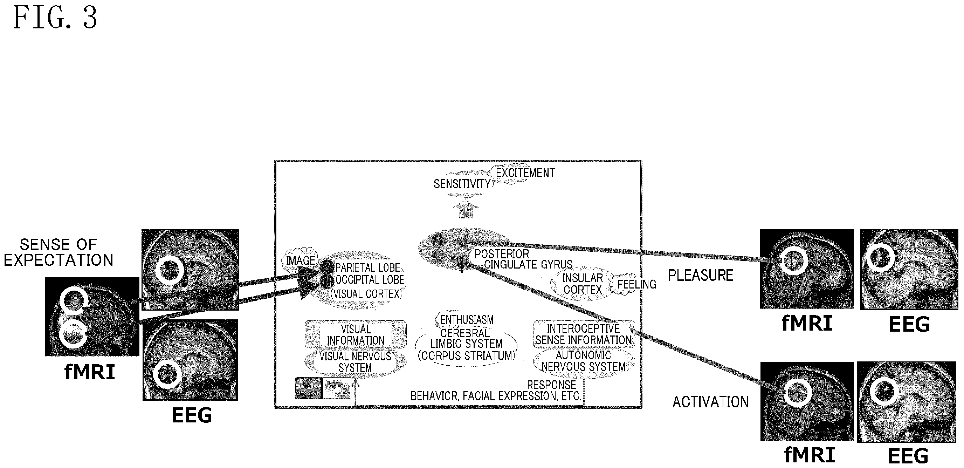

FIG. 3 shows regions of interest relevant to the axes of the multi-axis sensitivity model, together with the results of fMRI and EEG measurements on the cerebral responses related to the axes. An fMRI image and an EEG image, which are related to the "pleasure/displeasure" axis or the "activation/deactivation" axis in FIG. 3, respectively represent a difference (change) between signals obtained in a pleasant state and an unpleasant state, and a difference (change) between signals obtained in an active state and an inactive state. The fMRI image related to the "sense of expectation" axis is obtained in a pleasant image expectation state, and the EEG images respectively represent a difference between signals obtained in a pleasant image expectation state and those obtained in an unpleasant image expectation state.

As shown in FIG. 3, the results of the fMRI and EEG measurements indicate that cingulate gyrus is active when the subject feels "pleasant/unpleasant" and "active/inactive." It is also indicated that parietal lobe and visual cortex are active when the subject feels the "sense of expectation."

The regions of interest related to the axes of the multi-axis sensitivity model shown in FIG. 3 have been found through observations and experiments of the cerebral responses under various different conditions using fMRI and EEG. The observations and experiments will be specifically described below.

(1) Cerebral responses in Pleasant/Unpleasant State

First of all, a pleasant image (e.g., an image of a cute baby seal) and an unpleasant image (e.g., an image of hazardous industrial wastes), extracted from International Affective Picture System (IAPS), were presented to 27 participants to observe their cerebral responses when they were in a pleasant/unpleasant state.

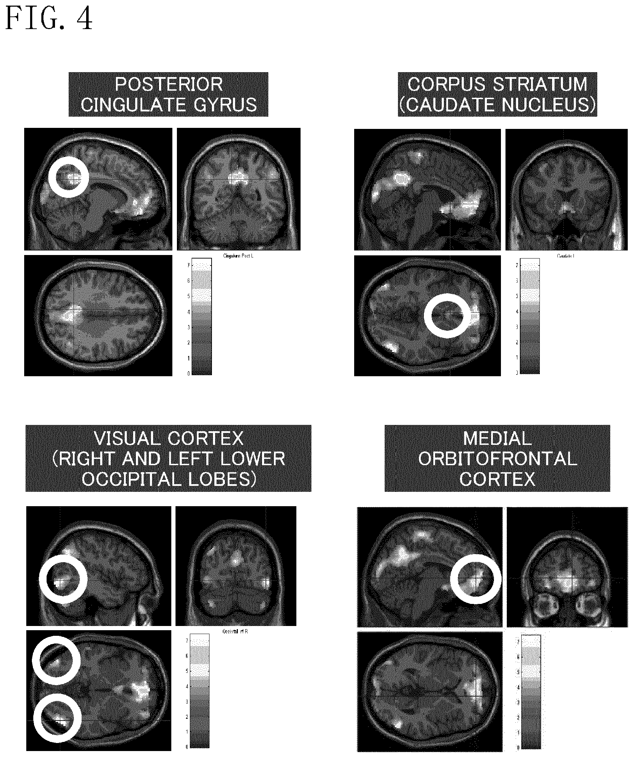

FIG. 4 shows various fMRI images (sagittal, coronal, and horizontal sections) of the brain in the pleasant state. In FIG. 4, regions that responded more significantly in a pleasant state (when the participant saw the pleasant image) than in an unpleasant state (when the participant saw the unpleasant image) are marked with circles. As can be seen from FIG. 4, posterior cingulate gyrus, visual cortex, corpus striatum, and orbitofrontal area are activated in the pleasant state.

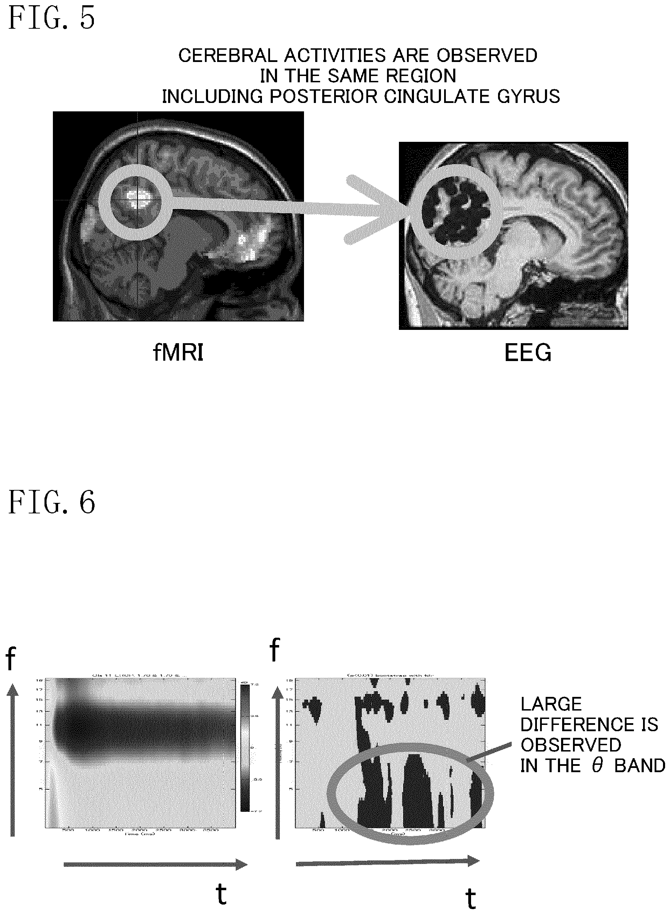

FIG. 5 shows an fMRI image, and a sagittal section of the brain on which EEG signal sources are plotted, both of which were obtained in the pleasant state. In FIG. 5, regions that responded more significantly in the pleasant state than in the unpleasant state are marked with circles. As can be seen from FIG. 5, the measurement results by fMRI and the measurement results by EEG show the cerebral activities in the same region including the posterior cingulate gyrus in the pleasant state. According to the results, the region including the cingulate gyrus can be identified as a region of interest related to the pleasant/unpleasant state.

FIG. 6 shows a result of a time-frequency analysis that was carried out on a signal of the EEG signal source in the region of interest (the posterior cingulate gyrus in the pleasant state). FIG. 6 shows, on the left, a result of a time-frequency analysis that was carried out on a signal of the EEG signal source in the region of interest (the posterior cingulate gyrus in the pleasant state). FIG. 6 shows, on the right, a difference between signals obtained in the pleasant state and those obtained in the unpleasant state. In the right graph of FIG. 6, dark-colored parts indicate that the difference is large. The results of the EEG measurement reveal that responses in the θ bands of the region of interest are involved in the pleasant state.

(2) Cerebral Responses in Active/Inactive State

First of all, an active image (e.g., an image of appetizing sushi) and an inactive image (e.g., an image of a castle stood in a quiet rural area), extracted from IAPS, were presented to 27 participants to observe their cerebral responses when they were in an active/inactive state.

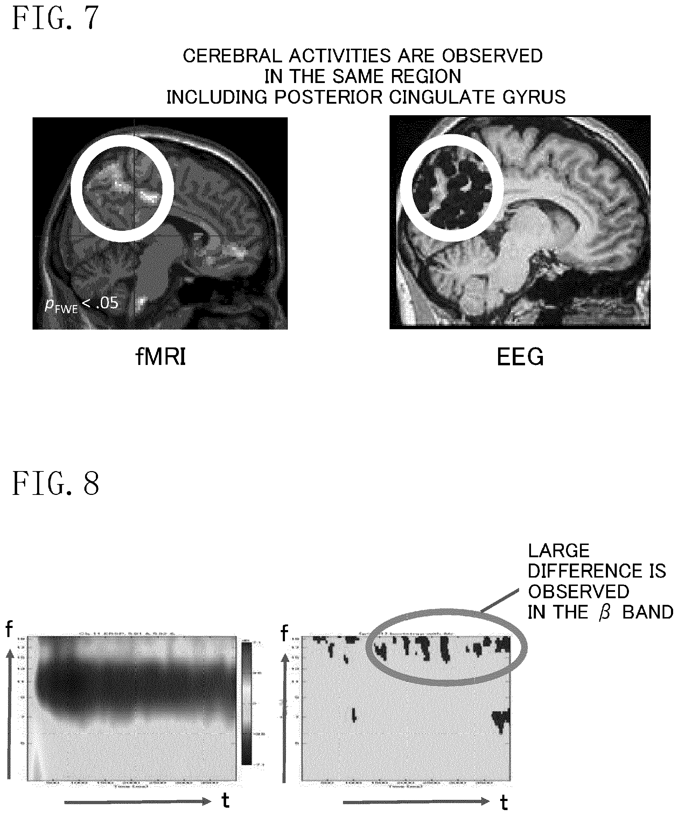

FIG. 7 shows an fMRI image, and a sagittal section of the brain on which EEG signal sources are plotted, both of which were obtained in the active state. In FIG. 7, regions that responded more significantly in the active state (when the participant saw the active image) than in the inactive state (when the participant saw the inactive image) are marked with circles. As can be seen from FIG. 7, the measurement results by fMRI and the measurement results by EEG show the cerebral activities in the same region including the posterior cingulate gyrus in the active state. According to the results, the region including the cingulate gyrus can be identified as a region of interest related to the active/inactive state.

FIG. 8 shows a result of a time-frequency analysis that was carried out on a signal of the EEG signal source in the region of interest (the posterior cingulate gyrus in the active state). FIG. 8 shows, on the left, a result of a time-frequency analysis that was carried out on a signal of the EEG signal source in the region of interest (the posterior cingulate gyrus in the active state). FIG. 8 shows, on the right, a difference between signals obtained in the active state and those obtained in the inactive state. In the right graph of FIG. 8, dark-colored parts indicate that the difference is large. The results of the EEG measurement reveal that responses in the β bands of the region of interest were involved in the active state.

(3) Cerebral Responses in Sense of Expectation

First of all, an experiment is carried out in which 27 participants are presented with stimulus images that will evoke their emotions to evaluate the feeling states of those participants who are viewing those images. As the stimulus images, 80 emotion-evoking color images, extracted from IAPS, are used. Of those 80 images, 40 are images that would evoke pleasure ("pleasant images") and the other 40 are images that would evoke displeasure ("unpleasant images").

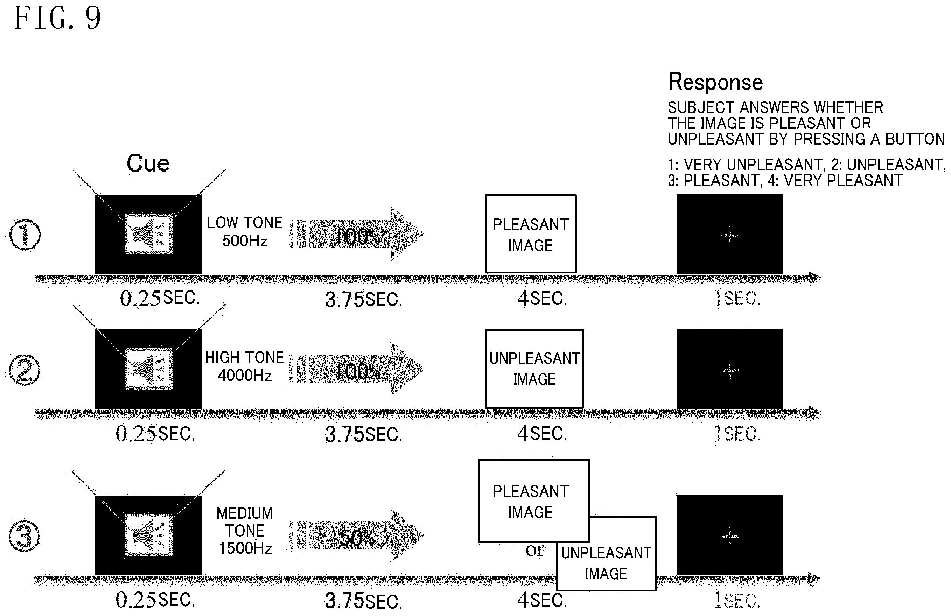

FIG. 9 illustrates generally how to carry out the experiment of presenting the participants with those pleasant/unpleasant stimulus images. Each of those stimulus images will be presented for only 4 seconds to the participants 3.75 seconds after a short tone (Cue) has been emitted for 0.25 seconds. Then, the participants are each urged to answer, by pressing the button, whether they have found the image pleasant or unpleasant. In this experiment, a pleasant image is presented to the participants every time a low tone (with a frequency of 500 Hz) has been emitted; an unpleasant image is presented to the participants every time a high tone (with a frequency of 4,000 Hz) has been emitted; and either a pleasant image or an unpleasant image is presented at a probability of 50% after a medium tone (with a frequency of 1,500 Hz) has been emitted.

In this experiment, that 4-second interval between a point in time when any of these three types of tones is emitted and a point in time when the image is presented is a period in which the participants expect what will happen next (i.e., presentation of either a pleasant image or an unpleasant image in this experiment). Their cerebral activities are observed during this expectation period. For example, when a low tone is emitted, the participants are in the state of "pleasant image expectation" in which they are expecting to be presented with a pleasant image. On the other hand, when a high tone is emitted, the participants are in the state of "unpleasant image expectation" in which they are expecting to be presented with an unpleasant image. Meanwhile, when a medium tone is emitted, the participants are in a "pleasant/unpleasant unexpectable state" in which they are not sure which of the two types of images will be presented, a pleasant image or an unpleasant image.

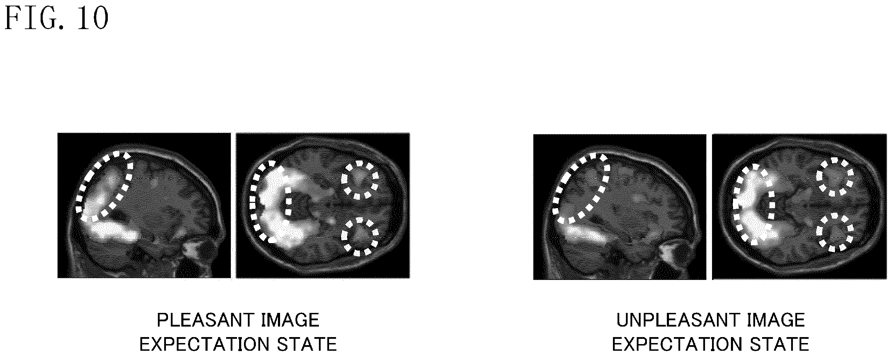

FIG. 10 shows fMRI images (representing sagittal and horizontal sections) of a subject's brain in the pleasant image expectation state and in the unpleasant image expectation state. As indicated clearly by the dotted circles in FIG. 10, it can be seen that according to fMRI, brain regions including the parietal lobe, visual cortex, and insular cortex are involved in the pleasant image expectation and unpleasant image expectation.

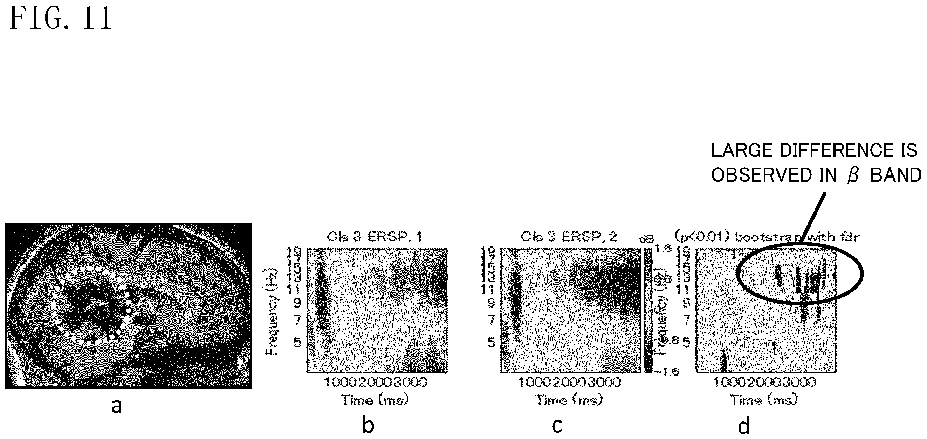

FIGS. 11a to 11d show the results of EEG measurement. FIG. 11a shows a sagittal section of a subject's brain, with a dotted circle added to a region that responded more significantly in the pleasant image expectation state than in the unpleasant image expectation state. FIG. 11b shows a result of a time-frequency analysis that was carried out on a signal of the EEG signal source in the region of interest (a region of the parietal lobe in the pleasant image expectation state). FIG. 11c shows a result of a time-frequency analysis that was carried out on a signal of the EEG signal source in the region of interest (a region of the parietal lobe in the unpleasant image expectation state). FIG. 11d shows the difference between the signals obtained in the pleasant image expectation state and the unpleasant image expectation state. In FIG. 11d, the region with a significant difference between them is encircled. Other regions had no difference. These EEG measurement results reveal that reactions in the β bands of the parietal lobe were involved in the pleasant image expectation.

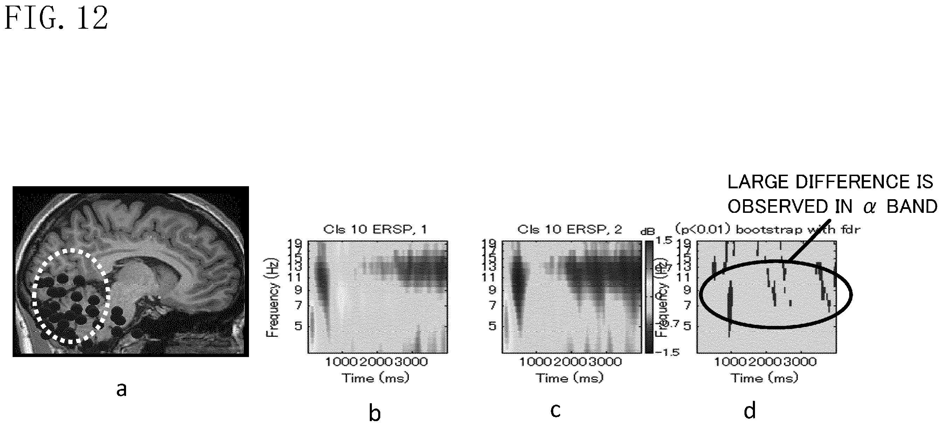

FIG. 12 shows the results of EEG measurement. FIG. 12a shows a sagittal section of a subject' brain, with a dotted circle added to a region that responded more significantly in the pleasant image expectation state than in the unpleasant image expectation state. FIG. 12b shows a result of a time-frequency analysis that was carried out on a signal of the EEG signal source in the region of interest (a region of the visual cortex in the pleasant image expectation state). FIG. 12c shows a result of a time-frequency analysis that was carried out on a signal of the EEG signal source in the region of interest (a region of the visual cortex in the unpleasant image expectation state). FIG. 12d shows the difference between the signals obtained in the pleasant image expectation state and the unpleasant image expectation state. In FIG. 12d, the region with a significant difference between them is encircled. Other regions had no difference. These EEG measurement results reveal that reactions in the α bands of the visual cortex were involved in the pleasant image expectation.

3. Visualization of Sensitivity

It has already been described that the sensitivity is represented using the multi-axis sensitivity model including three axes: the pleasure/displeasure axis; the activation/deactivation axis; and the sense of expectation (time) axis. The next challenge is how the sensitivity is specifically visualized and digitized to link the sensitivity with the construction of BEI.

The present inventors have found that the three axes of the sensitivity are not independent, but correlated with each other, and that it is necessary to obtain actual measurements of the axes and specify the relationship among the axes contributing to the sensitivity. Based on these findings, the present inventors have integrated a subjective psychological axis of the sensitivity and a cerebral physiological index in the following manner for visualization of the sensitivity.

$$\text{Sensitivity} = [\text{Subjective Psychological Axis}] \times [\text{Cerebral Physiological Index}]$$

$$= a \times \text{EEG}_{\text{pleasure}} + b \times \text{EEG}_{\text{activation}} + c \times \text{EEG}_{\text{sense of expectation}} \dots (\text{Formula 1})$$

where the subjective psychological axis represents weighting coefficients (a, b, c) of the axes, and the cerebral physiological index represents the values ($\text{EEG}_{\text{pleasure}}$, $\text{EEG}_{\text{activation}}$, $\text{EEG}_{\text{sense of expectation}}$) of the axes based on the results of EEG measurement.

It will be described below how to determine the subjective psychological axis and how to select the cerebral physiological index.

A. Determination of Subjective Psychological Axis

A contribution ratio, i.e., weighting, of each axis using the subjective psychological axis of the sensitivity can be determined in the following manner.

(1) The experiment of presenting the participants (27 male and female students) with the pleasant/unpleasant stimulus images is carried out as described above. Each participant is urged to make a self-evaluation of the sensitivity state of the brain during a 4-second interval (expectation state) between a point in time when the tone is emitted and a point in time when the image is presented.

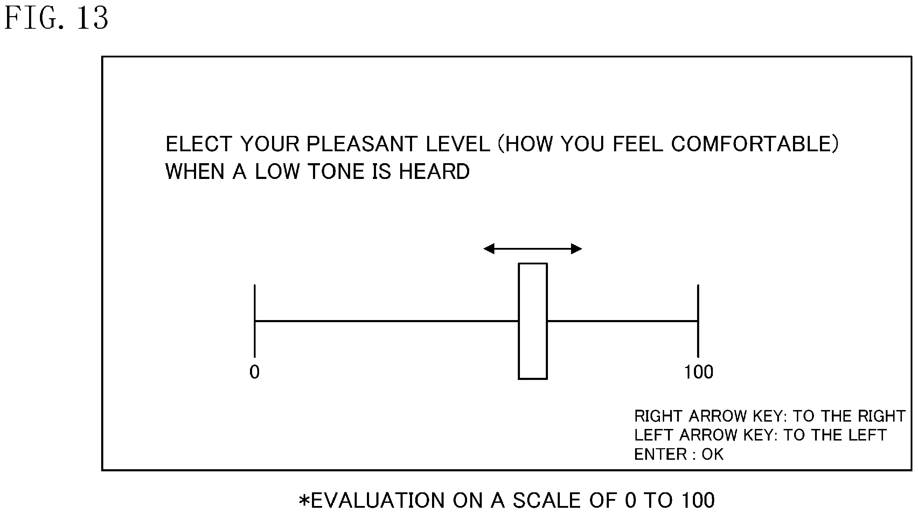

(2) The participants are urged to make an evaluation of an exhilaration (sensitivity) level, a pleasure level (pleasure axis), an activity level (activation axis), and a sense of expectation level (sense of expectation axis) on a scale of 101 from 0 to 100 using Visual Analog Scale (VAS) under three different conditions (in the pleasant image expectation state, the unpleasant image expectation state, and the pleasant/unpleasant unexpectable state). FIG. 13 shows an example of the self-evaluation for the determination of the subjective psychological axis, illustrating how the pleasure level is evaluated when a low tone is emitted (in the pleasant image expectation state). Each participant moves the cursor between 0 and 100 for the evaluation. As a result of the evaluation, for example, subjective evaluation values of: exhilaration = 73; pleasure = 68; activation = 45; and sense of expectation = 78 are obtained from one of the participants in the pleasant image expectation state.

(3) Coefficients of the subjective psychological axis are calculated through linear regression based on the subjective evaluation values obtained from all the participants under the three conditions. As a result, the following sensitivity evaluation formula based on the subjective psychological axis is obtained.

$$\text{Sensitivity} = 0.38 \times \text{Subjective}_{\text{pleasure}} + 0.11 \times \text{Subjective}_{\text{activation}} + 0.51 \times$$

$$\text{Subjective}_{\text{sense of expectation}} \ldots \text{(Formula 2)}$$

where the subjective$_{\text{pleasure}}$, the subjective$_{\text{activation}}$, and the subjective$_{\text{sense ofexpectation}}$ are values of the pleasure level, activation level, and sense of expectation level evaluated by the participants.

(4) The subjective$_{\text{pleasure}}$, subjective$_{\text{activation}}$, and subjective$_{\text{sense of expectation}}$ of the subjective psychological axis respectively correspond to the EEG$_{\text{pleasure}}$, EEG$_{\text{activation}}$, and EEG$_{\text{sense of expectation}}$ of the cerebral physiological index. Thus, the weighting coefficients of the axes of the subjective psychological axis calculated through the linear regression of the subjective evaluation values can be used as weighting coefficients of the EEG$_{\text{pleasure}}$, EEG$_{\text{activation}}$, and EEG$_{\text{sense of expectation}}$ of the cerebral physiological index. If the weighting coefficients of the axes obtained from Formula 2 are applied to Formula 1, the sensitivity can be represented by the following formula using the EEG$_{\text{pleasure}}$, the EEG$_{\text{activation}}$, and the EEG$_{\text{sense of expectation}}$ which are measured from moment to moment.

$$\text{Sensitivity} = 0.38 \times \text{EEG}_{\text{pleasure}} + 0.11 \times \text{EEG}_{\text{activation}} + 0.51 \times \text{EEG}_{\text{sense of}}$$

$$_{\text{expectation}} \ldots \text{(Formula 3)}$$

Specifically, the sensitivity can be visualized into numerical values by Formula 3.

B. Selection of Cerebral Physiological Index

The cerebral physiological index is an estimated value of each axis of the multi-axis sensitivity model calculated from the EEG measurement results. Since the cerebral activities are different among individuals, it is necessary to measure the EEG of each individual before real-time evaluation of the sensitivity so that independent components of his or her electroencephalogram and the frequency bands thereof are identified.

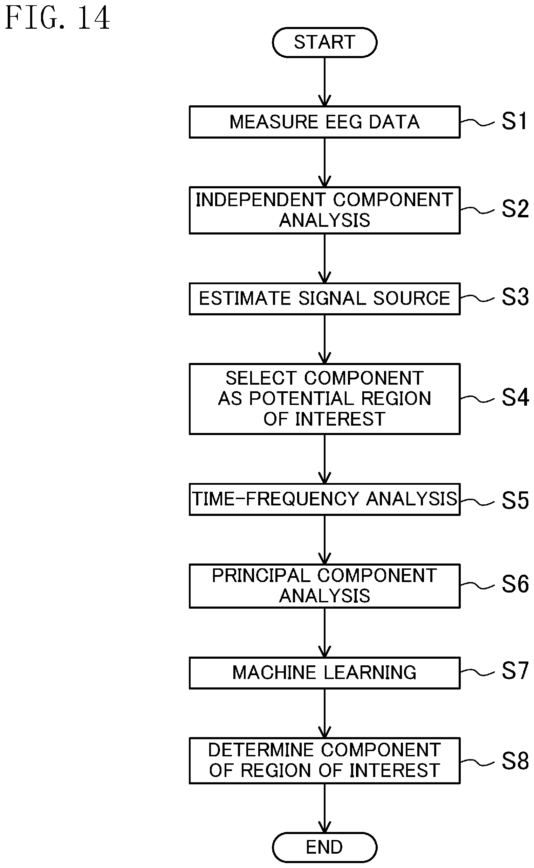

First, it will be described how to identify the frequency band used for measuring the electroencephalogram when the subject feels pleasant/unpleasant, active/inactive, and a sense of expectation. FIG. 14 shows a flow chart for identification of the independent components of the electroencephalogram in the region of interest and their frequency bands.

For example, an image which evokes pleasure/displeasure is presented as a visual stimulus to a subject, and an electroencephalogram signal induced by the stimulus is measured (step S1). Noise derived from blink, movement of eyes, and myoelectric potential (artifact) is removed from the measured electroencephalogram signal.

An independent component analysis (ICA) is performed on the measured electroencephalogram signal to extract independent components (signal sources) (step S2). For example, when the electroencephalogram is measured with 32 channels, the corresponding number of independent components, i.e., 32 independent components, are extracted. As a result of the independent component analysis of the measured electroencephalogram, the positions of the signal sources are identified (step S3).

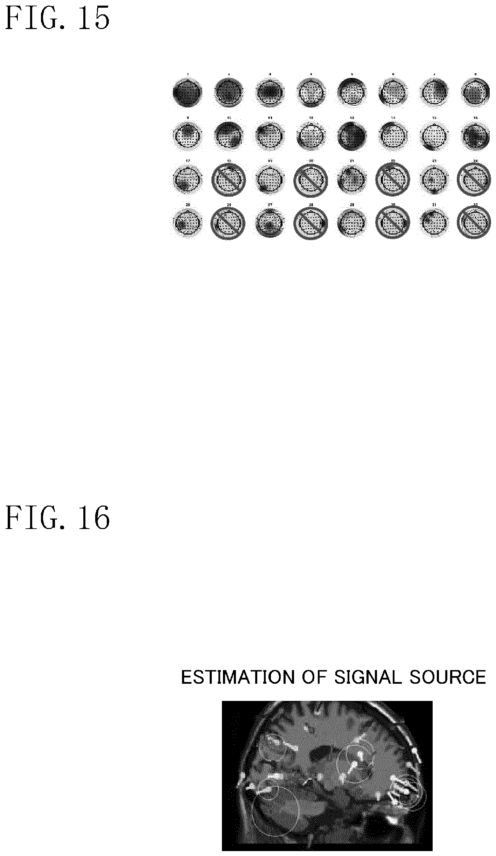

FIG. 15 shows components (electroencephalogram topographic images) representing signal intensity distributions of the independent components extracted through the independent component analysis carried out on the electroencephalogram signal in step S2. FIG. 16 shows a sagittal section of the brain on which estimated positions of the signal sources of the independent signal components are plotted.

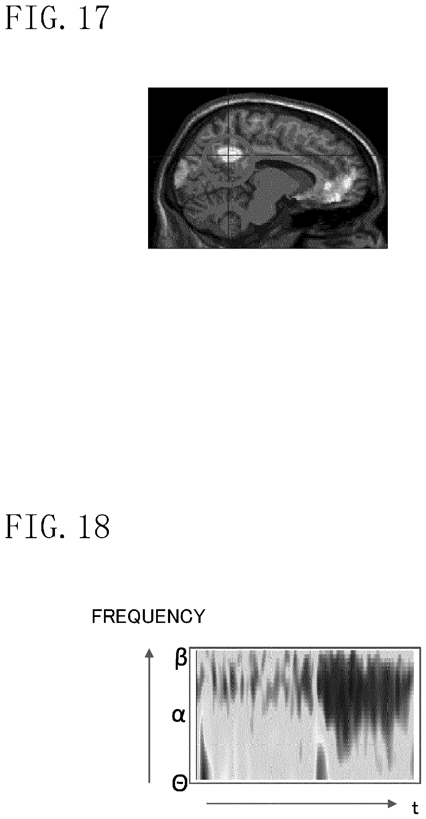

In addition to the measurement of the electroencephalogram, measurement by fMRI is also carried out. FIG. 17 shows an fMRI image obtained when the subject is in the pleasant/unpleasant state. As indicated by a circle in FIG. 17, cingulate gyrus is involved in the pleasant/unpleasant state.

Through the fMRI measurement performed separately, it has been revealed that the cingulate gyrus is involved in the "pleasant" state, for example. Thus, if the independent component related to the "pleasant" state is a target to be selected, the signal sources (independent components) present around the cingulate gyrus can be selected as potential regions of interest (step S4). For example, 10 independent components are selected out of the 32 independent components.

With respect to each of the signals (e.g., 10 independent components) from the signal sources as the potential regions of interest, a time-frequency analysis is performed to calculate a power value at each time point and each frequency point (step S5). For example, 20 frequency points are set at each of 40 time points to calculate the power value at 800 points in total.

FIG. 18 shows a result of a time-frequency analysis that was carried out on the signals of the EEG signal sources in step S5. In the graph of FIG. 18, a vertical axis represents the frequency, and a horizontal axis the time. The frequency $\beta$ is the highest, $\alpha$ is the second highest, and $\theta$ is the lowest. The intensity of the gray in the graph corresponds to the signal intensity. The result of the time-frequency analysis is actually shown in color, but the graph of FIG. 18 is shown in gray scale for convenience.

Then, a principal component analysis (PCA) is performed on each of the independent components gained through time-frequency decomposition, thereby narrowing each independent component into a principal component based on time and frequency bands (step S6). In this way, the number of features is narrowed. For example, the features at the 800 points are dimensionally reduced to 40 principal components.

Discrimination learning is carried out on the narrowed time-frequency principal components using sparse logistic regression (SLR) (step S7). Thus, the principal component (time frequency) which contributes to the discrimination of the axis (e.g., the pleasure/displeasure axis) of the independent component (signal source) is detected. For example, in measuring the subject's "pleasure," it is determined that the $\theta$ band of the signal source in the region of interest is relevant. Further, the discrimination accuracy in the frequency band of the independent component can be calculated, for example, the accuracy of the discrimination between two choices of pleasure and displeasure is 70%.

Based on the calculated discrimination accuracy, the independent component and its frequency band with a significant discrimination rate is identified (step S8). Thus, among the 10 independent components as the potential regions of interest, for example, the most potential independent component and its frequency band are selected.

The procedure for measuring the pleasant/unpleasant feeling has been described above. In the similar procedure, the independent components of the electroencephalogram in the region of interest and their frequency bands are identified for the measurement of the active/inactive feeling and the sense of expectation. The results of the measurements reveal that the $\beta$ band of the region of interest is involved in the activation/deactivation, and the $\theta$ to $\alpha$ bands are involved in the sense of expectation.

The results obtained through the above-described procedure are applied as a spatial filter in the next real-time sensitivity evaluation.

In steps S3 and S4 described above, the signal sources of all the independent components are estimated, and then the signal sources (independent components) are narrowed based on the fMRI information. Alternatively, steps S3 to S7 may be carried out without using the fMRI information, and in the last step S8, some independent components (signal sources) may be selected, using the fMRI information, from the independent components significantly contributing to the discrimination, and then, a single independent component which is the most contributory to the discrimination may be selected from them. The results are the same in this procedure.

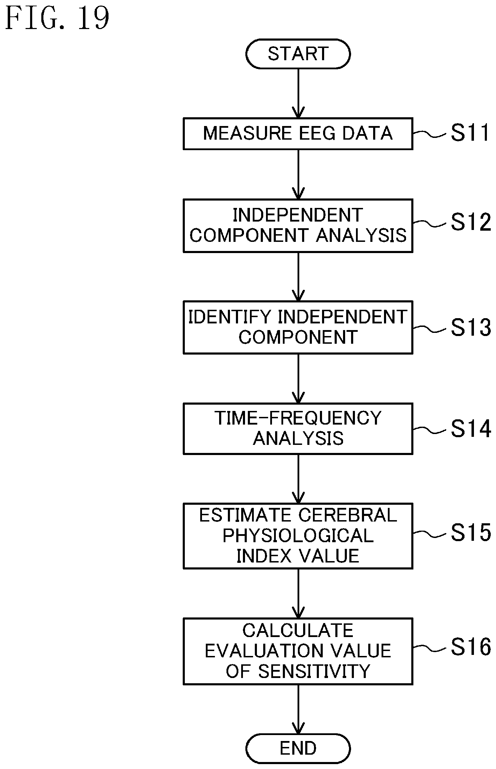

It will be described below a procedure of real-time sensitivity evaluation through estimation of the subject's cerebral activities that vary from moment to moment using the frequency bands of the independent components identified in the above-described procedure. FIG. 19 shows a flow chart of real-time sensitivity evaluation using the electroencephalogram.

The subject's electroencephalogram is measured to extract electroencephalogram information (cerebral activities at each channel) in real time (step S11). Noise derived from blink, movement of eyes, and myoelectric potential (artifact) is removed from the EEG signals measured from the channels.

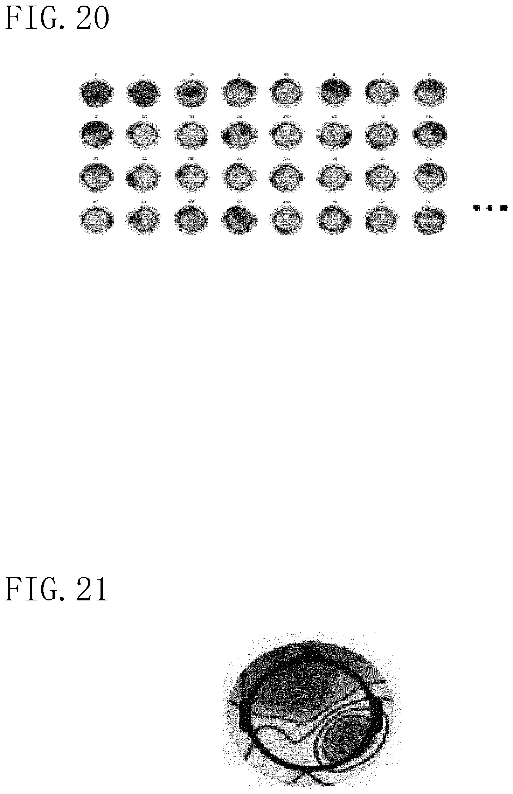

An independent component analysis is performed on the measured electroencephalogram signals to extract independent components (signal sources) (step S12). For example, when the electroencephalogram is measured with 32 channels, the corresponding number of independent components, i.e., 32 independent components, are extracted. FIG. 20 shows components (electroencephalogram topographic images) representing signal intensity distribution in each independent component extracted through the independent component analysis of the electroencephalogram signal in step S12.

Among 32 independent components thus extracted, the independent component related to the region of interest is specified (step S13). In this step, the target independent component has already been identified through the procedure shown by the flow chart of FIG. 14. Thus, the target component is easily identified. FIG. 21 shows the component identified as the independent component related to the region of interest.

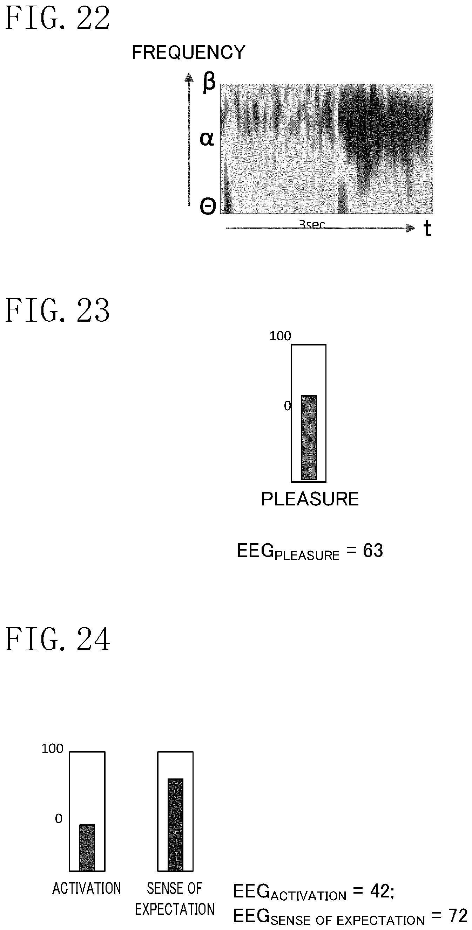

Then, a time-frequency analysis is carried out on the identified independent component to calculate a time-frequency spectrum (step S14). FIG. 22 shows the result of the time-frequency analysis carried out on the identified independent component.

It has been determined in the measurement of the subject's "pleasure" that the $\theta$ band of the independent component (the signal source in the region of interest) is the relevant frequency band. Thus, a value of the pleasure/displeasure axis (cerebral physiological index value) at a certain point of time is estimated from the signal intensity of the spectrum in the θ band (step S15). The cerebral physiological index value is represented as a numeric value of 0 to 100, for example. FIG. 23 schematically shows the estimated value of the pleasure/displeasure axis. For example, as shown in FIG. 23, the value $EEG_{pleasure} = 63$ is estimated as the value of the pleasure/displeasure axis.

In the same manner as the above-described procedure for measuring the subject's pleasure/displeasure, the cerebral physiological index value is estimated in the measurement of the subject's activation/deactivation and the sense of expectation. FIG. 24 schematically shows the estimated values of the activation/deactivation axis and the sense of expectation axis. For example, as shown in FIG. 24, the value $EEG_{activation} = 42$ is estimated as the value of the activation/deactivation axis, and the value $EEG_{sense\ of\ expectation} = 72$ is estimated as the value of the sense of expectation axis (time axis).

The estimated values of the cerebral physiological index are substituted into Formula 3 to calculate the evaluation value of the sensitivity (step S16). For example, if the values $EEG_{pleasure} = 63$, $EEG_{activation} = 42$, and $EEG_{sense\ of\ expectation} = 72$ are estimated, the evaluation value of the sensitivity is calculated as 65.28.

4. Sensitivity Evaluation Method using EEG

Another sensitivity evaluation method using EEG will be described below. The following has been described in Japanese Patent Application No. 2015-204963 to which the present application claims priority.

The axes of the multi-axis sensitivity model can be evaluated by cerebral activity data represented in accordance with the electroencephalogram. For example, suppose that the values of the cerebral activity data related to the pleasure/displeasure obtained from the electroencephalogram are $EEGV_1$, $EEGV_2$, ..., and $EEGV_i$, and the coefficients of these values are $v_1$, $v_2$, ..., and $v_i$, the evaluation value Valence of the first axis of the multi-axis sensitivity model of FIG. 2 can be represented as:

$$Valence = v_1 \times EEGV_1 + v_2 \times EEGV_2 + ... + v_i \times EEGV_i$$

Suppose that the values of the cerebral activity data related to the activation/deactivation obtained from the electroencephalogram are $EEGA_1$, $EEGA_2$, ..., and $EEGA_j$, and the coefficients of these values are $a_1$, $a_2$, ..., and $a_j$, the evaluation value Arousal of the second axis of the multi-axis sensitivity model of FIG. 2 can be represented as:

$$Arousal = a_1 \times EEGA_1 + a_2 \times EEGA_2 + ... + a_j \times EEGA_j$$

Suppose that the values of the cerebral activity data related to the time obtained from the electroencephalogram are $EEGT_1$, $EEGT_2$, ..., and $EEGT_k$, and the coefficients of these values are $t_1$, $t_2$, ..., and $t_k$, the evaluation value Time of the third axis of the multi-axis sensitivity model of FIG. 2 can be represented as:

$$Time = t_1 \times EEGT_1 + t_2 \times EEGT_2 + ... + t_k \times EEGT_k$$

Then, suppose that the coefficients of these axes are a, b, c, respectively, the evaluation value Emotion of the sensitivity can be represented as:

$$Emotion = a \times Valence + b \times Arousal + c \times Time$$

The coefficients v, a, and t used for calculating the evaluation values of the axes of the multi-axis sensitivity model, and the coefficients a, b, c used for calculating the evaluation value Emotion of the sensitivity may be any value, and be set according to the purpose. For example, to increase (amplify) the evaluation value, the coefficients may be set to be 1 or more. To decrease (attenuate) the evaluation value, the coefficients may be set to be 0 or more and less than 1. In particular, the coefficients a, b, and c used for calculating the evaluation value Emotion of the sensitivity may be set within a range of 0 to 1, and to be 1 in total, so that the evaluation values of the axes of the multi-axis sensitivity model can be weighted-summed. In this way, the sensitivity can be evaluated by biasing the evaluation values of the axes in accordance with the significance or contribution of the axes of the multi-axis sensitivity model.

In FIG. 2, the sensitivity is represented using the three axes. Alternatively, more axes may be used for modeling the sensitivity. The pleasure/displeasure axis and the activation/deactivation axis described above are mere examples of the axes, and the sensitivity may be represented by a multi-axis model using different axes.

(1) Experimental Examples

An experiment was performed to examine the relationship between the subject's pleasure/displeasure and the electroencephalogram. The experiment has been described briefly with reference to FIG. 9, and is not repeated below.

At least 64 electrodes were attached to a head of each subject to obtain electroencephalogram signals when he or she was watching presented stimulus images. The stimulus images were presented 200 times or less to stabilize the electroencephalogram signals. Sampling of the electroencephalogram signals was performed at a rate of 1000 Hz or more.

A highpass filter of 1 Hz was used for every electroencephalogram signal.

The electroencephalogram signal measured by the electrodes may include, in addition to the potential variation associated with the cerebral (cortical) activities, an artifact such as blink and myoelectric potential, and external noise such as noise of a commercial power supply. The potential variation associated with the cerebral (cortical) activities is very weak, and lower than the artifact and the external noise, and thus, has a very low S/N ratio. Therefore, in order to extract a signal which may probably reflect pure cerebral responses from the measured electroencephalogram signals, the noise is removed as much as possible.

For each of the subjects, the independent component analysis was performed on the electroencephalogram signal after the noise removal. Then, for each of the obtained independent components, the positions of dipoles were estimated.

Further, for each of the independent components obtained through the independent component analysis, a principal component analysis was carried out for dimensionality reduction. Then, all the independent components after the dimensionality reduction were formed into 16 clusters by k-means clustering.

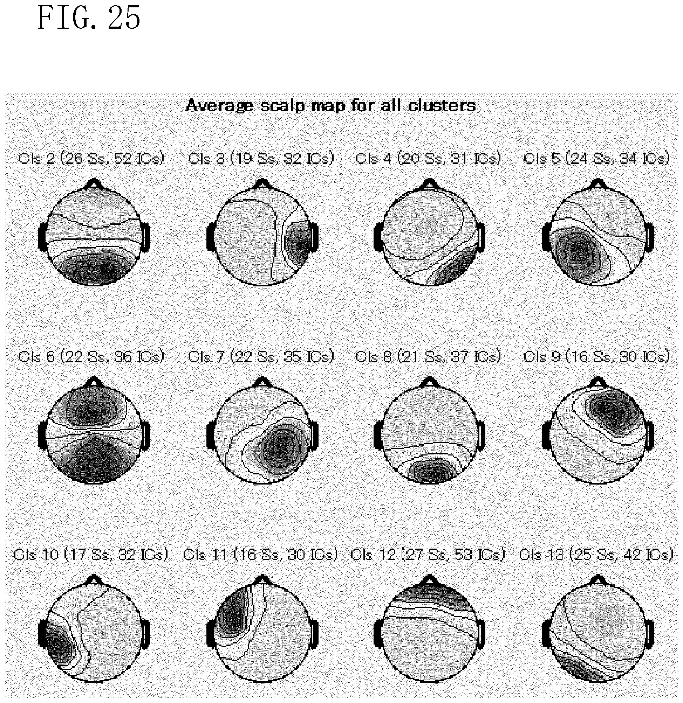

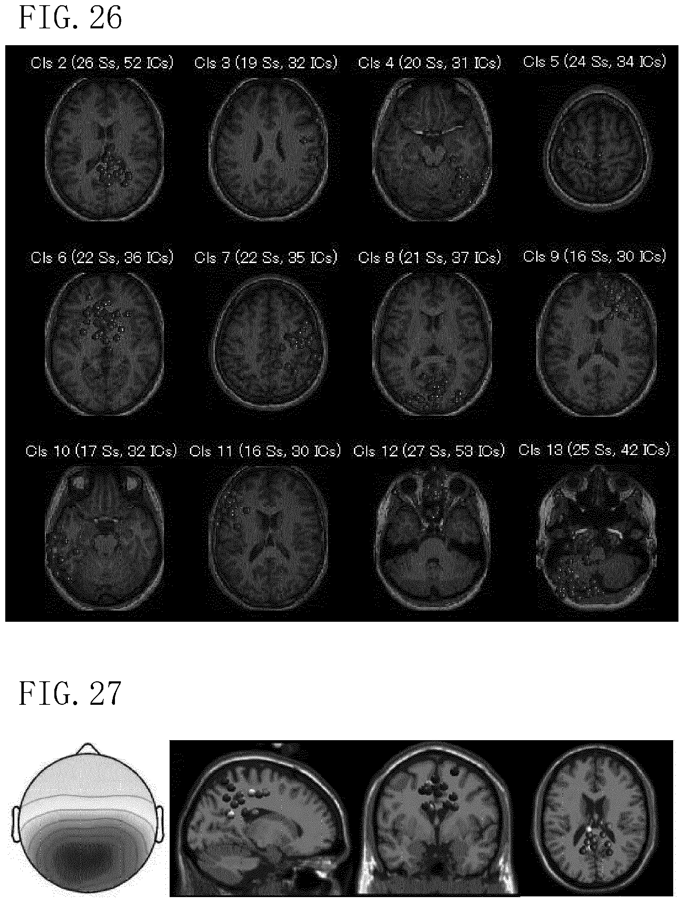

FIG. 25 shows electroencephalogram topographic images of the 16 clusters. FIG. 26 shows brain images of the 16 clusters on each of which the estimated positions of dipoles are plotted.

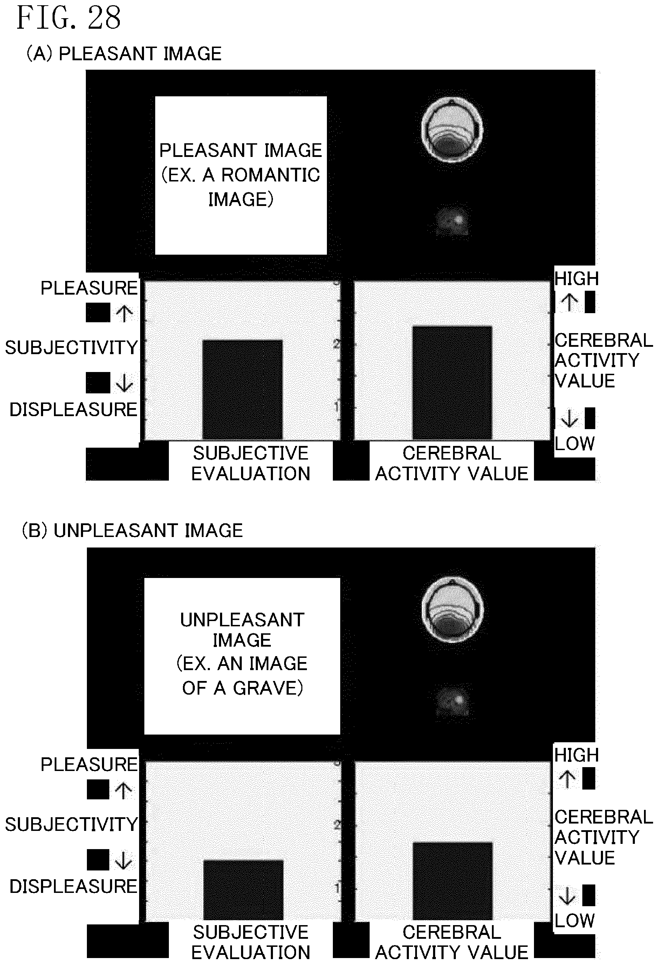

Among the 16 clusters, there was a single cluster which showed a significant difference ($p < 0.05$) between signals during pleasant stimulus presentation and unpleasant stimulus presentation, and a high correlation between the cerebral activity data and the subjective evaluation value of the subject. FIG. 27 shows an electroencephalogram topographic image of the cluster which had a significant difference between signals during the pleasant stimulus presentation and the unpleasant stimulus presentation, and brain images on each of which the estimated positions of dipoles are plotted. FIG. 28 shows a correlation between the subjective evaluation value and the cerebral activities when the subject watches (a) a pleasant image and (b) an unpleasant image. The cluster of FIG. 27 showed a high correlation between the subjective evaluation value and the cerebral activities in both cases when the subject saw the pleasant image and the unpleasant image. Thus, this experiment confirmed that at least a region around posterior cingulate gyrus is deeply involved in the human feelings or emotions such as pleasure/displeasure.

It is also expected that the activities of the same or different portion of the brain are deeply involved in the activation/deactivation and the time.

(2) Embodiments

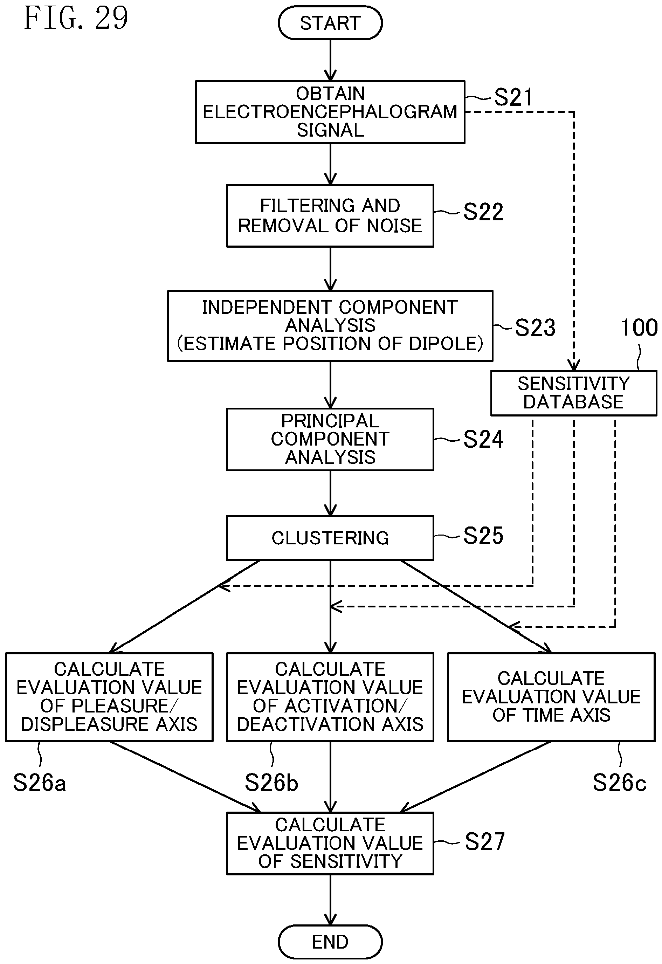

Based on the findings obtained through the experiment, the sensitivity evaluation method of the present disclosure using the electroencephalogram can be performed in the following manner. FIG. 29 shows a flow chart of the sensitivity evaluation method using the electroencephalogram according to an embodiment of the present disclosure. The following processing flow can be conducted with a general-purpose computer such as a PC.

With a certain stimulus (object) presented to the subject, electroencephalogram signals of the subject are obtained (step S21). For example, 64 electrodes are attached to the subject's head to obtain the electroencephalogram signals from 64 channels.

The electroencephalogram signals thus obtained are filtered, and subjected to noise removal (step S22). The filtering is performed with, for example, a highpass filter of 1 Hz. The noise removal includes removal of an artifact, and disturbance noise such as noise of a commercial power supply.

After the filtering and the noise removal, an independent component analysis is performed on the electroencephalogram signals. Then, for each of the obtained independent components, the positions of dipoles are estimated (step S23). Specifically, from the electroencephalogram measured on the subject's scalp, a major activity source in the brain is estimated as a dipole (electric doublet). For example, the electroencephalogram signals from the 64 channels are decomposed to 64 independent components (estimated positions of the dipoles).

For each of the independent components obtained through the independent component analysis, a principal component analysis is carried out for dimensionality reduction (step S24).

Then, all the independent components after the dimensionality reduction are formed into a predetermined number of clusters (e.g., 16 clusters) by k-means clustering (step S25).

A cluster which represents the cerebral activities reflecting the pleasure/displeasure is selected from the obtained clusters, and an evaluation value Valence of the pleasure/displeasure axis of the multi-axis sensitivity model is calculated from components contained in the selected cluster (data of cerebral activities from the signal sources at the estimated dipole positions) (step S26a).

Another cluster which represents the cerebral activities reflecting the activation/deactivation is selected from the obtained clusters, and an evaluation value Arousal of the activation/deactivation axis of the multi-axis sensitivity model is calculated from components contained in the selected cluster (data of cerebral activities from the signal sources at the estimated dipole positions) (step S26b).

Still another cluster which represents the cerebral activities reflecting the time is selected from the obtained clusters, and an evaluation value Time of the time axis of the multi-axis sensitivity model is calculated from components contained in the selected cluster (data of cerebral activities from signal sources at the estimated dipole positions) (step S26c).

In steps S26a, S26b, and S26c, the clusters may be selected with reference to a sensitivity database 100. The sensitivity database 100 stores a huge amount of electroencephalogram signals and subjective evaluation values of multiple subjects obtained through trials that have been carried out so far. Through the estimation of the dipole positions and the clustering using the huge data stored in the sensitivity database 100, the clusters to be selected in steps S26a, S26b, and S26c can be identified.

To enlarge the sensitivity database 100, in one preferred embodiment, the electroencephalogram signals obtained in step S21 and the subjective evaluation values of the subjects with respect to the presented stimuli may also be recorded in the sensitivity database 100.

The evaluation values calculated in steps S26a, S26b, and S26c are synthesized to calculate the sensitivity evaluation value Emotion (step S27).

5. Sensitivity Evaluation Method using fMRI

Another sensitivity evaluation method using fMRI will be described below. The following has been described in Japanese Patent Application No. 2015-204969 to which the present application claims priority.

The axes of the multi-axis sensitivity model can be evaluated by cerebral activity data represented in accordance with fMRI. For example, suppose that the values of the cerebral activity data related to the pleasure/displeasure obtained by fMRI are $fMRIV_1$, $fMRIV_2$, ..., and $fMRIV_i$, and the coefficients of these values are $v_1$, $v_2$, ..., and $v_i$, the evaluation value Valence of the first axis of the multi-axis sensitivity model of FIG. 2 can be represented as:

$$Valence = v_1 \times fMRIV_1 + v_2 \times fMRIV_2 + ... + v_i \times fMRIV_i$$

Suppose that the values of the cerebral activity data related to the activation/deactivation obtained by fMRI are $fMRIA_1$, $fMRIA_2$, ..., and $fMRIA_j$, and the coefficients of these values are $a_1$, $a_2$, ..., and $a_j$, the evaluation value Arousal of the second axis of the multi-axis sensitivity model of FIG. 2 can be represented as:

$$Arousal = a_1 \times fMRIA_1 + a_2 \times fMRIA_2 + ... + a_j \times fMRIA_j$$

Suppose that the values of the cerebral activity data related to the time obtained by fMRI are $fMRIT_1$, $fMRIT_2$, ..., and $fMRIT_k$, and the coefficients of these values are $t_1$, $t_2$, ..., and $t_k$, the evaluation value Time of the third axis of the multi-axis sensitivity model of FIG. 2 can be represented as:

$$Time = t_1 \times fMRIT_1 + t_2 \times fMRIT_2 + ... + t_k \times fMRIT_k$$

Then, suppose that the coefficients of these axes are a, b, c, respectively, the evaluation value Emotion of the sensitivity can be represented as:

$$Emotion = a \times Valence + b \times Arousal + c \times Time$$

The coefficients v, a, and t used for calculating the evaluation values of the axes of the multi-axis sensitivity model, and the coefficients a, b, and c used for calculating the evaluation value Emotion of the sensitivity may be any value, and be set according to the purpose. For example, to increase (amplify) the evaluation value, the coefficients may be set to be 1 or more. To decrease (attenuate) the evaluation value, the coefficients may be set to be 0 or more and less than 1. In particular, the coefficients a, b, and c used for calculating the evaluation value Emotion of the sensitivity may be set within a range of 0 to 1, and to be 1 in total, so that the evaluation values of the axes of the multi-axis sensitivity model can be weighted-summed. In this way, the sensitivity can be evaluated by biasing the evaluation values of the axes in accordance with the significance or contribution of the axes of the multi-axis sensitivity model.

In FIG. 2, the sensitivity is represented using the three axes. Alternatively, more axes may be used for modeling the sensitivity. The pleasure/displeasure axis and the activation/deactivation axis described above are mere examples of the axes, and the sensitivity may be represented by a multi-axis model using different axes.

(1) Experimental Examples

According to reports of prior studies, an experiment using emotion-evoking images has revealed that insula and amygdala in the brain are involved in expectation of displeasure, and character traits of a human (Simmons et al., 2006; Schuerbeek et al., 2014). However, such prior studies are somewhat limited because only a few images were used, or comparison between signals in pleasant image presentation and unpleasant image presentation was not performed. To generalize the results of the experiment, it is of great importance to obtain findings in line with various circumstances. For this purpose, an experiment was carried out using various types of stimulus images to examine the relationship between the individual's character traits and the state of activities of a particular portion of the brain (level of activation) in the expectation of pleasure/displeasure. The experiment has been described briefly with reference to FIG. 9, and is not repeated below.

As the character traits of the subjects, a "harm avoidance" score was used. Each of the subjects was urged to fill a questionnaire of temperament and character inventory (TCI), which is one of character traits tests, to obtain the "harm avoidance" score. The higher harm avoidance score indicates that the subject has a higher tendency to show pessimistic concern about future events, or exhibit a passive avoidance behavior toward expectation of undesirable situations (e.g., the subject tries to avoid a certain aversive stimulus by keeping a distance from it or taking no action).

For the fMRI measurement on the subjects, a 3TMRI apparatus was used. The stimulus images were presented 120 times to stabilize the signals derived from the cerebral activities.

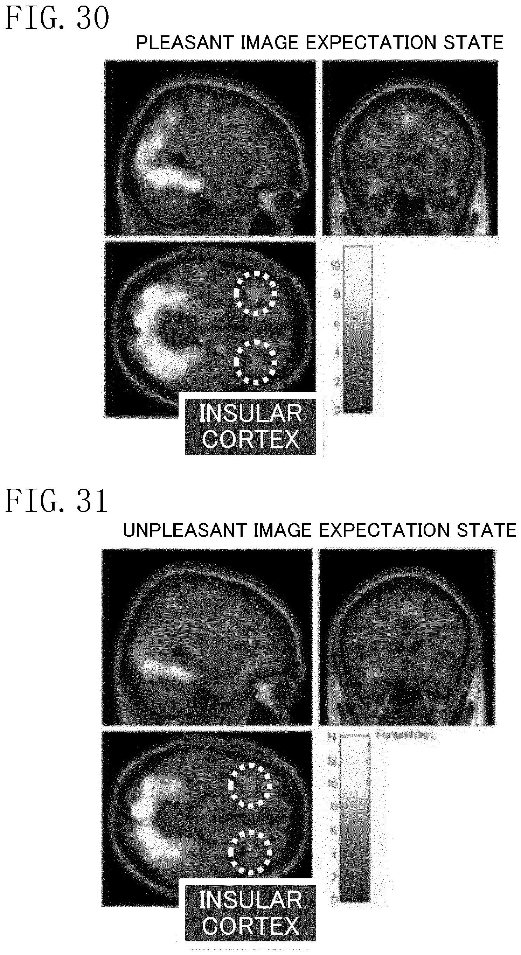

FIG. 30 shows images of the brain in the pleasant image expectation state, i.e., after the low tone was emitted. FIG. 31 shows images of the brain in the unpleasant image expectation state, i.e., after the high tone was emitted. In each of the pleasant image expectation state and the unpleasant image expectation state, an increase in the level of activation was observed in a brain region including insular cortex.

It was also confirmed that, from the comparison between the level of activation of the region including the insular cortex in the pleasant image expectation state and that in the unpleasant image expectation state, the subject having the higher harm avoidance score showed the relatively higher level of activation in the unpleasant image expectation state. Specifically, it was confirmed that the region including the insular cortex has a region having a positive correlation with the harm avoidance score.

It is also expected that the activities of the same or different portion of the brain are deeply involved in the pleasure/displeasure and the activation/deactivation.

(2) Embodiments

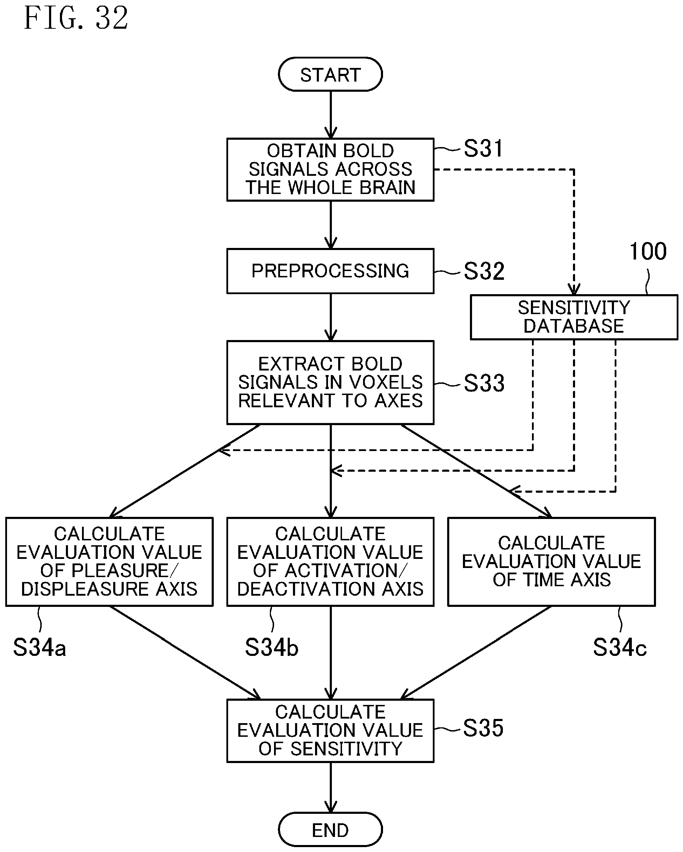

Based on the findings obtained through the experiment, the sensitivity evaluation method of the present disclosure using fMRI can be performed in the following manner. FIG. 32 shows a flow chart of the sensitivity evaluation method using fMRI according to an embodiment of the present disclosure. The following processing flow can be conducted with a general-purpose computer such as a PC.

With a certain stimulus (object) presented to the subject, BOLD signals across the whole brain of the subject are obtained by fMRI (step S31).

Spatiotemporal preprocessing is performed on the obtained whole-brain BOLD signals (step S32).

Of the preprocessed whole-brain BOLD signals, some in voxels representing the cerebral activities reflecting the pleasure/displeasure, some in voxels representing the cerebral activities reflecting the activation/deactivation, and some in voxels representing the cerebral activities reflecting the time are extracted (step S33).

From the BOLD signals in the voxels representing the cerebral activities reflecting the pleasure/displeasure, an evaluation value Valence of the pleasure/displeasure axis of the multi-axis sensitivity model is calculated (step S34a).

From the BOLD signals in the voxels representing the cerebral activities reflecting the activation/deactivation, an evaluation value Arousal of the activation/deactivation axis of the multi-axis sensitivity model is calculated (step S34b).

From the BOLD signals in the voxels representing the cerebral activities reflecting the time, an evaluation value Time of the time axis of the multi-axis sensitivity model is calculated (step S34c).

In step S33, the voxels can be selected with reference to a sensitivity database 100. The sensitivity database 100 stores a huge amount of fMRI data and subjective evaluation values of multiple subjects obtained through trials that have been carried out so far. With a help of the huge data stored in the sensitivity database 100, a cluster to be selected in step S33 is identified.

To enlarge the sensitivity database 100, in one preferred embodiment, the whole-brain BOLD signals obtained in step S31 and the subjective evaluation values of the subjects with respect to the presented stimuli may also be recorded in the sensitivity database 100.

The evaluation values calculated in steps S34a, 34b, and 34c are synthesized to calculate the sensitivity evaluation value Emotion (step S35).

In one preferred embodiment, the calculation of the evaluation values in steps S34a, 34b, and 34c may reflect the character traits of the subject. For example, it has been confirmed that a person with a high harm avoidance tendency shows significant cerebral responses to a negative event. Specifically, as compared with a person with a low harm avoidance tendency, a person with a high harm avoidance tendency may possibly show a higher absolute value of the sensitivity evaluation value Emotion as a whole. Thus, the evaluation value Emotion of a person with a high harm avoidance tendency becomes harder to interpret than that of a person with a low harm avoidance tendency. For example, suppose that an event is slightly unwelcome (e.g., the evaluation value "Emotion" is about -20), and another event is very unwelcome (e.g., the evaluation value "Emotion" is -100 or less), to a person with a low harm avoidance tendency. To both of these events, a person with a high harm avoidance tendency would show excessive cerebral responses (the evaluation value "Emotion" of -100 or less in each event), which makes it difficult to understand the difference between the responses to the events. Thus, for example, in the calculation of the evaluation value Valence of the pleasure/displeasure axis in step S34a, the coefficient $v_1$, $v_2$, ..., and $v_i$ may be set to be 1 or less, for example, so that the evaluation value Valence can be attenuated if the subject has a low harm avoidance tendency. It is assumed that such an attenuation process makes the interpretation of the sensitivity evaluation value Emotion of a person with a high harm avoidance tendency as easy as the interpretation of the evaluation value "Emotion" of a person with a low harm avoidance tendency.

As can be seen in the foregoing, embodiments have just been described as examples of the technique disclosed in the present invention. For this purpose, accompanying drawings and detailed description have been provided.

The components illustrated on the accompanying drawings and described in the detailed description include not only essential components that need to be used to overcome the problem, but also other unessential components that do not have to be used to overcome the problem. Therefore, such unessential components should not be taken for essential ones, simply because such unessential components are illustrated in the drawings or mentioned in the detailed description.

The above embodiments, which have been described as examples of the technique of the present disclosure, may be altered or substituted, to which other features may be added, or from which some features may be omitted, within the range of claims or equivalents to the claims.

INDUSTRIAL APPLICABILITY

A sensitivity evaluation method according to the present disclosure can quantitatively evaluate the sensitivity using cerebral physiological information such as an electroencephalogram. Thus, the present disclosure is useful as a fundamental technology for realizing BEI that links humans to objects.

**Claims**

1. A method for evaluating sensitivity, the method comprising:

   extracting cerebral physiological information items related to axes of a multi-axis sensitivity model from regions of interest respectively relevant to pleasure/displeasure, activation/deactivation, and a sense of expectation, the axes including a pleasure/displeasure axis, an activation/deactivation axis, and a sense-of-expectation axis; and

   evaluating the sensitivity using the cerebral physiological information items of the axes of the multi-axis sensitivity model.

2. The method of claim 1, wherein
   in the evaluation of the sensitivity, correlation among the axes of the multi-axis sensitivity model is obtained to evaluate the sensitivity using the correlation and the cerebral physiological information items of the axes of the multi-axis sensitivity model.

3. The method of claim 1 or 2, wherein

the regions of interest relevant to the pleasure/displeasure and the activation/deactivation are a region including cingulate gyrus.

4. The method of claim 1 or 2, wherein
the region of interest relevant to the sense of expectation is a region including parietal lobe, occipital lobe, and insular cortex.

5. The method of any one of claims 1 to 4, wherein
the cerebral physiological information items are derived from electroencephalogram signals,
the extraction of the cerebral physiological information items includes steps performed for each of the axes of the multi-axis sensitivity model, the steps including:

measuring electroencephalogram signals of a subject;
extracting a plurality of independent components through an independent component analysis performed on the measured electroencephalogram signals;
calculating a time-frequency spectrum through a time-frequency analysis performed on one of the plurality of independent components associated with the concerned axis; and
estimating, as the cerebral physiological information item, a cerebral physiological index value from a spectrum intensity, of the time-frequency spectrum, in a frequency band of interest associated with the concerned axis.

6. The method of claim 5, wherein
in the estimation of the cerebral physiological index value, the region of interest is identified using BOLD signals measured by fMRI.

7. The method of claim 5 or 6, wherein
a frequency band of the region of interest relevant to the pleasure/displeasure is a $\theta$ band.

8. The method of claim 5 or 6, wherein
a frequency band of the region of interest relevant to the activation/deactivation is a $\beta$ band.

9. The method of claim 5 or 6, wherein
a frequency band of the region of interest relevant to the sense of expectation is $\theta$ to $\alpha$ bands.

10. The method of any one of claims 1 to 4, wherein
the cerebral physiological information items are derived from BOLD signals measured by fMRI,
the extraction of the cerebral physiological information items includes steps performed for each of the axes of the multi-axis sensitivity model, the steps including:

obtaining BOLD signals across a whole brain of a subject by fMRI;
selecting, from the obtained BOLD signals, BOLD signals associated with the concerned axis; and
estimating, from the selected BOLD signals, a cerebral physiological index value as the cerebral physiological information item.

11. A method for evaluating sensitivity, the method comprising:

extracting cerebral physiological information items related to axes of a multi-axis sensitivity model from regions of interest respectively relevant to pleasure/displeasure, activation/deactivation, and a sense of expectation, the axes including a pleasure/displeasure axis, an activation/deactivation axis, and a sense-of-expectation axis; and
obtaining cerebral physiological index values ($EEG_{pleasure}$, $EEG_{activation}$, and $EEG_{sense\ of\ expectation}$) of the axes from the cerebral physiological information items of the axes of the multi-axis sensitivity model; and
evaluating the sensitivity by the following formula using a subjective psychological axis which is obtained from subjective statistical data of a subject and represents weighting coefficients (a, b, c) of the axes of the multi-axis sensitivity model:

$$\text{Sensitivity} = [\text{Subjective Psychological Axis}] \times [\text{Cerebral Physiological Index}]$$

$$= a \times \text{EEG}_{\text{pleasure}} + b \times \text{EEG}_{\text{activation}} + c \times \text{EEG}_{\text{sense of expectation}}$$

12. A method for evaluating sensitivity using an electroencephalogram, the method comprising:

obtaining electroencephalogram signals of a subject;
performing an independent component analysis on the obtained electroencephalogram signals to estimate the position of a dipole for each of the independent components;
performing a principal component analysis on the independent components obtained through the independent component analysis to dimensionally reduce cerebral activity data of the independent components;
forming clusters of the cerebral activity data of the dimensionally reduced independent components;
selecting, from the obtained clusters, a cluster representing cerebral activities respectively reflecting various feelings or emotions;
calculating evaluation values of the feelings or emotions from components included in the selected cluster; and
calculating an evaluation value of the sensitivity by synthesizing the calculated evaluation values of the feelings or emotions.

13. The method of claim 12, wherein
the sensitivity is represented by a multi-axis model having various feelings or emotions as axes, and
in the calculation of the evaluation value of the sensitivity, the evaluation value of the sensitivity is calculated through weighted summing of the evaluation values of the axes.

14. The method of claim 13, wherein
the sensitivity is represented by a triple-axis model.

15. The method of claim 13, wherein
the multi-axis sensitivity model includes at least an axis representing a pleasant/unpleasant feeling or emotion, and
cerebral activities reflecting the pleasant/unpleasant feeling or emotion are cerebral activities of a region around posterior cingulate gyrus.

16. A method for evaluating sensitivity by fMRI, the method comprising:

obtaining BOLD signals across a whole brain of a subject by fMRI;
selecting, from the obtained BOLD signals, BOLD signals in a voxel representing cerebral activities respectively reflecting various feelings or emotions;
calculating evaluation values of the feelings or emotions from the selected BOLD signals in the voxel; and
calculating an evaluation value of the sensitivity by synthesizing the calculated evaluation values of the feelings or emotions.

17. The method of claim 16, wherein
the sensitivity is represented by a multi-axis model having feelings or emotions as axes, and
in the calculation of the evaluation value of the sensitivity, the evaluation value of the sensitivity is calculated through weighted summing of the evaluation values of the axes.

18. The method of claim 17, wherein
the sensitivity is represented by a triple-axis model.

19. The method of claim 17, wherein
the multi-axis sensitivity model includes at least an axis representing a feeling or emotion of expectation of pleasure/expectation of displeasure, and
cerebral activities reflecting the feeling or emotion of expectation of pleasure/expectation of displeasure are cerebral activities of a region including insular cortex.

FIG. 1

HIGHER

LOWER

SENSITIVITY

FEELING

EMOTION

DAMASIO MODEL
SETH MODEL

# FIG. 2

MULTI-AXIS SENSITIVITY MODEL

FIG. 3

# FIG. 4

POSTERIOR CINGULATE GYRUS

CORPUS STRIATUM (CAUDATE NUCLEUS)

VISUAL CORTEX (RIGHT AND LEFT LOWER OCCIPITAL LOBES)

MEDIAL ORBITOFRONTAL CORTEX

## FIG. 5

CEREBRAL ACTIVITIES ARE OBSERVED
IN THE SAME REGION
INCLUDING POSTERIOR CINGULATE GYRUS

fMRI                    EEG

## FIG. 6

LARGE
DIFFERENCE IS
OBSERVED
IN THE $\theta$ BAND

# FIG. 7

CEREBRAL ACTIVITIES ARE OBSERVED
IN THE SAME REGION
INCLUDING POSTERIOR CINGULATE GYRUS

fMRI                    EEG

# FIG. 8

LARGE
DIFFERENCE IS
OBSERVED
IN THE $\beta$ BAND

# FIG. 9

EP 3 360 480 A1

Response

SUBJECT ANSWERS WHETHER
THE IMAGE IS PLEASANT OR
UNPLEASANT BY PRESSING A BUTTON

1: VERY UNPLEASANT, 2: UNPLEASANT,
3: PLEASANT, 4: VERY PLEASANT

Cue

① LOW TONE 500Hz  100%  PLEASANT IMAGE  +

0.25SEC.  3.75SEC.  4SEC.  1SEC.

② HIGH TONE 4000Hz  100%  UNPLEASANT IMAGE  +

0.25SEC.  3.75SEC.  4SEC.  1SEC.

③ MEDIUM TONE 1500Hz  50%  PLEASANT IMAGE or UNPLEASANT IMAGE  +

0.25SEC.  3.75SEC.  4SEC.  1SEC.

FIG. 10

UNPLEASANT IMAGE
EXPECTATION STATE

PLEASANT IMAGE
EXPECTATION STATE

FIG. 11

LARGE DIFFERENCE IS
OBSERVED IN $\beta$ BAND

a

Cls 3 ERSP, 1

Frequency (Hz)

1000 2000 3000
Time (ms)

b

Cls 3 ERSP, 2

dB
1.6

-1.6

1000 2000 3000
Time (ms)

c

(p<0.01) bootstrap with fdr

Frequency (Hz)

1000 2000 3000
Time (ms)

d

FIG. 12

LARGE DIFFERENCE IS
OBSERVED IN $\alpha$ BAND

a

b

c

d

FIG. 13

ELECT YOUR PLEASANT LEVEL (HOW YOU FEEL COMFORTABLE)
WHEN A LOW TONE IS HEARD

0                                                    100

RIGHT ARROW KEY: TO THE RIGHT
LEFT ARROW KEY: TO THE LEFT
ENTER : OK

*EVALUATION ON A SCALE OF 0 TO 100

EP 3 360 480 A1

## FIG. 14

```
        START

MEASURE EEG DATA          ~ S1

INDEPENDENT COMPONENT     ~ S2
      ANALYSIS

ESTIMATE SIGNAL SOURCE    ~ S3

  SELECT COMPONENT
AS POTENTIAL REGION       ~ S4
    OF INTEREST

TIME-FREQUENCY ANALYSIS   ~ S5

PRINCIPAL COMPONENT       ~ S6
      ANALYSIS

   MACHINE LEARNING       ~ S7

DETERMINE COMPONENT       ~ S8
OF REGION OF INTEREST

          END
```

FIG. 15

FIG. 16

ESTIMATION OF SIGNAL SOURCE

FIG. 17

FIG. 18

FREQUENCY

FIG. 19

```
        ┌─────────────┐
        │    START    │
        └─────────────┘
               │
               ▼
   ┌─────────────────────┐
   │   MEASURE EEG DATA  │───S11
   └─────────────────────┘
               │
               ▼
   ┌─────────────────────┐
   │     INDEPENDENT     │
   │  COMPONENT ANALYSIS │───S12
   └─────────────────────┘
               │
               ▼
   ┌─────────────────────┐
   │ IDENTIFY INDEPENDENT│
   │     COMPONENT       │───S13
   └─────────────────────┘
               │
               ▼
   ┌─────────────────────┐
   │   TIME-FREQUENCY    │
   │      ANALYSIS       │───S14
   └─────────────────────┘
               │
               ▼
   ┌─────────────────────┐
   │  ESTIMATE CEREBRAL  │
   │    PHYSIOLOGICAL    │───S15
   │     INDEX VALUE     │
   └─────────────────────┘
               │
               ▼
   ┌─────────────────────┐
   │     CALCULATE       │
   │  EVALUATION VALUE   │───S16
   │   OF SENSITIVITY    │
   └─────────────────────┘
               │
               ▼
        ┌─────────────┐
        │     END     │
        └─────────────┘
```

FIG. 20

FIG. 21

FIG. 22

FREQUENCY

$\beta$

$\alpha$

$\Theta$

3sec

t

FIG. 23

100

0

PLEASURE

$EEG_{PLEASURE} = 63$

FIG. 24

100

0

ACTIVATION    SENSE OF
              EXPECTATION

$EEG_{ACTIVATION} = 42;$

$EEG_{SENSE\ OF\ EXPECTATION} = 72$

# FIG. 25

Average scalp map for all clusters

Cls 2 (26 Ss, 52 ICs)   Cls 3 (19 Ss, 32 ICs)   Cls 4 (20 Ss, 31 ICs)   Cls 5 (24 Ss, 34 ICs)

Cls 6 (22 Ss, 36 ICs)   Cls 7 (22 Ss, 35 ICs)   Cls 8 (21 Ss, 37 ICs)   Cls 9 (16 Ss, 30 ICs)

Cls 10 (17 Ss, 32 ICs)   Cls 11 (16 Ss, 30 ICs)   Cls 12 (27 Ss, 53 ICs)   Cls 13 (25 Ss, 42 ICs)

## FIG. 26

Cls 2 (26 Ss, 52 ICs)  Cls 3 (19 Ss, 32 ICs)  Cls 4 (20 Ss, 31 ICs)  Cls 5 (24 Ss, 34 ICs)

Cls 6 (22 Ss, 36 ICs)  Cls 7 (22 Ss, 35 ICs)  Cls 8 (21 Ss, 37 ICs)  Cls 9 (16 Ss, 30 ICs)

Cls 10 (17 Ss, 32 ICs)  Cls 11 (16 Ss, 30 ICs)  Cls 12 (27 Ss, 53 ICs)  Cls 13 (25 Ss, 42 ICs)

## FIG. 27

# FIG. 28

(A) PLEASANT IMAGE

(B) UNPLEASANT IMAGE

FIG. 29

```
                    ┌──────────┐
                    │  START   │
                    └──────────┘
                         │
                         ▼
          ┌──────────────────────────┐
          │         OBTAIN           │──── S21
          │  ELECTROENCEPHALOGRAM    │
          │         SIGNAL           │
          └──────────────────────────┘
                         │
                         ▼
          ┌──────────────────────────┐
          │      FILTERING AND       │──── S22
          │    REMOVAL OF NOISE      │
          └──────────────────────────┘
                         │
                         ▼
          ┌──────────────────────────────┐
          │  INDEPENDENT COMPONENT       │──── S23
          │        ANALYSIS              │
          │ (ESTIMATE POSITION OF DIPOLE)│
          └──────────────────────────────┘
                         │                    ┌──────────────┐ 100
                         ▼                    │  SENSITIVITY │
          ┌──────────────────────────┐       │   DATABASE   │
          │        PRINCIPAL         │──── S24 └──────────────┘
          │        COMPONENT         │
          │        ANALYSIS          │
          └──────────────────────────┘
                         │
                         ▼
          ┌──────────────────────────┐
          │       CLUSTERING         │──── S25
          └──────────────────────────┘

  ┌────────────────┐  ┌────────────────┐  ┌────────────────┐
  │   CALCULATE    │  │   CALCULATE    │  │   CALCULATE    │
  │ EVALUATION VALUE│ │ EVALUATION VALUE│ │ EVALUATION VALUE│
  │  OF PLEASURE/  │  │ OF ACTIVATION/ │  │  OF TIME AXIS  │
  │DISPLEASURE AXIS│  │DEACTIVATION AXIS│ │                │
  └────────────────┘  └────────────────┘  └────────────────┘
       S26a                S26b                S26c
                         │
                         ▼
          ┌──────────────────────────┐
          │       CALCULATE          │──── S27
          │   EVALUATION VALUE       │
          │    OF SENSITIVITY        │
          └──────────────────────────┘
                         │
                         ▼
                    ┌──────────┐
                    │   END    │
                    └──────────┘
```

## FIG. 30

PLEASANT IMAGE EXPECTATION STATE

INSULAR CORTEX

## FIG. 31

UNPLEASANT IMAGE EXPECTATION STATE

INSULAR CORTEX

FIG. 32

```
                        ┌─────────┐
                        │  START  │
                        └─────────┘
                             │
                             ▼
                    ┌──────────────────┐
                    │   OBTAIN BOLD    │─── S31
                    │ SIGNALS ACROSS   │········
                    │ THE WHOLE BRAIN  │       :
                    └──────────────────┘       :
                             │                 :
                             ▼                 :
                    ┌──────────────────┐       :
                    │  PREPROCESSING   │── S32  :           100
                    └──────────────────┘       :            :
                             │          ┌──────────────────┐
                             │          │   SENSITIVITY    │
                             ▼          │    DATABASE      │
                    ┌──────────────────┐└──────────────────┘
                    │  EXTRACT BOLD    │── S33
                    │ SIGNALS IN VOXELS│
                    │ RELEVANT TO AXES │
                    └──────────────────┘
```

| CALCULATE EVALUATION VALUE OF PLEASURE/ DISPLEASURE AXIS | CALCULATE EVALUATION VALUE OF ACTIVATION/ DEACTIVATION AXIS | CALCULATE EVALUATION VALUE OF TIME AXIS |
|---|---|---|
| S34a | S34b | S34c |

```
                    ┌──────────────────┐
                    │    CALCULATE     │── S35
                    │ EVALUATION VALUE │
                    │  OF SENSITIVITY  │
                    └──────────────────┘
                             │
                             ▼
                        ┌─────────┐
                        │   END   │
                        └─────────┘
```

## INTERNATIONAL SEARCH REPORT

| International application No. |
| --- |
| PCT/JP2016/003712 |

A. CLASSIFICATION OF SUBJECT MATTER
*A61B5/16*(2006.01)i, *A61B5/0484*(2006.01)i, *A61B5/055*(2006.01)i

According to International Patent Classification (IPC) or to both national classification and IPC

B. FIELDS SEARCHED

Minimum documentation searched (classification system followed by classification symbols)
A61B5/16, A61B5/0484, A61B5/055

Documentation searched other than minimum documentation to the extent that such documents are included in the fields searched

| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
PubMed

C. DOCUMENTS CONSIDERED TO BE RELEVANT

| Category* | Citation of document, with indication, where appropriate, of the relevant passages | Relevant to claim No. |
| --- | --- | --- |
| X<br>Y<br>A | COI Program Kickoff Symposium (24 February 2014 (24.02.2014)) vision 2-1, YouTube, [online], published on 16 March 2014 (16.03.2014), [retrieval date 18 October 2016 (18.10.2016)], Internet<URL:https://www.youtube.com/watch?v=_9 _jmDRlscw&list=PLwlAbCcz-l4tiqcPpxljpT98JTLnwVk fr&index=9>, particularly, 8:45 to 11:55 | 1-3<br>10,16-18<br>4-9,11-15,19 |
| X<br>Y<br>A | 'Center of KANSEI Innovation Nurturing Mental Wealth', COI Program Kickoff Symposium Shiryo, 26 February 2014 (26.02.2014) | 1-3<br>10,16-18<br>4-9,11-15,19 |

| ☒ Further documents are listed in the continuation of Box C. | ☐ See patent family annex. |
| --- | --- |

\*    Special categories of cited documents:
"A"  document defining the general state of the art which is not considered    to be of particular relevance
"E"  earlier application or patent but published on or after the international filing date
"L"  document which may throw doubts on priority claim(s) or which is cited to establish the publication date of another citation or other special reason (as specified)
"O"  document referring to an oral disclosure, use, exhibition or other means
"P"  document published prior to the international filing date but later than the priority date claimed

"T"  later document published after the international filing date or priority date and not in conflict with the application but cited to understand the principle or theory underlying the invention
"X"  document of particular relevance; the claimed invention cannot be considered novel or cannot be considered to involve an inventive step when the document is taken alone
"Y"  document of particular relevance; the claimed invention cannot be considered to involve an inventive step when the document is combined with one or more other such documents, such combination being obvious to a person skilled in the art
"&"  document member of the same patent family

| Date of the actual completion of the international search<br>    19 October 2016 (19.10.16) | Date of mailing of the international search report<br>    01 November 2016 (01.11.16) |
| --- | --- |
| Name and mailing address of the ISA/<br>    Japan Patent Office<br>    3-4-3,Kasumigaseki,Chiyoda-ku,<br>    Tokyo 100-8915,Japan | Authorized officer<br><br>Telephone No. |

Form PCT/ISA/210 (second sheet) (January 2015)

**INTERNATIONAL SEARCH REPORT**

| International application No. |
| --- |
| PCT/JP2016/003712 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | Ayaka KOBAYASHI, "A study of estimation of human emotion induced by images using fMRI", Kochi University of Technology Gakushi Gakui Ronbun, [online], archival date 26 February 2015 (26.02.2015), [retrieval date 18 October 2016 (18.10.2016)], Internet<URL:https://web.archive.org/web/20150226072229/http://www.kochi-tech.ac.jp/library/ron/2013/2013info/1140328.pdf> | 10,16-18 |
| A | Yuto HONMA, "Experiment of Fuzzy Emotion Inference Used Emotion Measurement System Based on Electroencephalogram", Japan Society for Fuzzy Theory and Intelligent Informatics Fuzzy System Symposium Koen Ronbunshu, 23rd Fuzzy System Symposium, 14 January 2009 (14.01.2009), Session ID: WD3-4 (pages 226 to 229) | 1-19 |
| A | Gi CHIN, "Noha o Mochiita Gairai Shigeki ni Taisuru Shinri Jotai no Hyoka", Shizuoka University REpository, [online], 2014.12, [retrieval date 18 October 2016 (18.10.2016)], Internet<URL: http://ir.lib.shizuoka.ac.jp/bitstream/10297/8787/1/K0862.pdf>, particularly, pages 23 to 25, 29, 82 to 83 | 1-19 |
| A | Naoko UTSUNOMIYA, "Construction of Pleasantness Estimation Matrix by the Correlation Coefficients of EEG", The Transactions of the Institute of Electrical Engineers of Japan. C, a Publication of Electronics, Information and Systems Society, vol.122, 2002, pages 309 to 310 | 1-19 |
| A | HUSTER, Rene J., Methods for Simultaneous EEG-fMRI: An Introductory Review, The Journal of Neuroscience, 2012, Vol.32, No.18, pp.6053-6060 | 1-19 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003058298 A
- JP 2011150408 A
- JP 2014115913 A
- JP 2006095266 A
- JP 2011120824 A
- JP 2005058449 A
- JP 2015204963 A
- JP 2015204969 A